# EUROPEAN PATENT APPLICATION

(11) **EP 4 067 376 A1**
(43) Date of publication of application: **05.10.2022**
(21) Application number: 21166096.4
(22) Date of filing: 30.03.2021
(51) Int. Cl.: C07K 16/18, A61K 39/00

(54) **ANTI-PLA2G1B MONOCLONAL ANTIBODIES AND USES THEREOF**

(71) Applicant: Diaccurate, 75008 Paris (FR)
(72) Inventor: THEZE, Jacques, 75007 Paris (FR); POTHLICHET, Julien, 92310 Sevres (FR)
(74) Representative: Cabinet Becker et Associés

(57) **Abstract**

The present invention relates to anti-PLA2G1B monoclonal antibodies and the uses thereof. The invention more particularly discloses new monoclonal antibodies which can bind and inhibit human or simian PLA2G1B. The invention is particularly useful for detecting, monitoring or treating an immunodeficiency or an associated disease caused, for example, by HIV, SIV or SHIV.

SEQUENCE LISTING

<110>  DIACCURATE

<120>  ANTI-PLA2G1B MONOCLONAL ANTIBODIES AND USES THEREOF

<130>  B3517EP00

<160>  38

<170>  PatentIn version 3.5

<210>  1
<211>  111
<212>  PRT
<213>  Artificial Sequence

<220>
<223>  amino acid sequence of mAb1 variable light chain region (mAb1-VL)

<400>  1

Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Asp Asn Tyr
            20                  25                  30

Gly Ile Ser Phe Met His Trp Tyr Gln Gln Lys Pro Gly Gln Pro Pro
        35                  40                  45

Lys Leu Leu Ile Tyr Arg Ala Ser Asn Leu Glu Ser Gly Ile Pro Ala
    50                  55                  60

Arg Phe Ser Gly Ser Gly Ser Arg Thr Asp Phe Thr Leu Thr Ile Asn
65                  70                  75                  80

Pro Val Glu Thr Asp Asp Val Ala Thr Tyr Tyr Cys Gln Gln Ser Ser
                85                  90                  95

Arg Asp Leu Phe Thr Phe Gly Gly Gly Thr Lys Leu Glu Ile Lys
            100                 105                 110

<210>  2
<211>  107
<212>  PRT
<213>  Artificial Sequence

<220>
<223>  amino acid sequence of mAb1 constant light chain region Ck

<400>  2

Arg Ala Asp Ala Ala Pro Thr Val Ser Ile Phe Pro Pro Ser Ser Glu
1               5                   10                  15

Gln Leu Thr Ser Gly Gly Ala Ser Val Val Cys Phe Leu Asn Asn Phe
            20                  25                  30

Tyr Pro Arg Asp Ile Asn Val Lys Trp Lys Ile Asp Gly Ser Glu Arg
        35                  40                  45

Gln Asn Gly Val Leu Asn Ser Trp Thr Asp Gln Asp Ser Lys Asp Ser
    50                  55                  60

Thr Tyr Ser Met Ser Ser Thr Leu Thr Leu Thr Lys Asp Glu Tyr Glu
65                  70                  75                  80

Arg His Asn Ser Tyr Thr Cys Glu Ala Thr His Lys Thr Ser Thr Ser
                85                  90                  95

Pro Ile Val Lys Ser Phe Asn Arg Asn Glu Cys
            100                 105

<210>  3
<211>  333
<212>  DNA
<213>  Artificial Sequence

<220>
<223>  nucleotide sequence of mAb1 variable light chain region (mAb1-VL)

<400>  3
gacattgtgc tgacccaatc tccagcttct ttggctgtgt ctctagggca gagggccacc      60

atctcctgca gagccagcga aagtgttgat aattatggca ttagttttat gcactggtac     120

cagcagaaac cgggacagcc acccaaactc ctcatctatc gtgcatccaa cctagaatct     180

gggatccctg ccaggttcag tggcagtggg tctaggacag acttcaccct caccattaat     240

cctgtggaga ctgatgatgt tgcaacctat tactgtcagc aaagtagtag ggatctgttc     300

acgttcggag gggggaccaa gctggaaata aaa                                  333

<210>  4
<211>  321
<212>  DNA
<213>  Artificial Sequence

<220>
<223>  nucleotide sequence of mAb1 constant light chain region Ck

<400>  4
cgggctgatg ctgcaccaac tgtatccatc ttcccaccat ccagtgagca gttaacatct      60

ggaggtgcct cagtcgtgtg cttcttgaac aacttctacc ccagagacat caatgtcaag     120

tggaagattg atggcagtga acgacaaaat ggtgtcctga acagttggac tgatcaggac     180

agcaaagaca gcacctacag catgagcagc accctcacat tgaccaagga cgagtatgaa     240

cgacataaca gctatacctg tgaggccact cacaagacat caacttcacc catcgtcaag 300

agcttcaaca ggaatgagtg t 321

<210> 5
<211> 121
<212> PRT
<213> Artificial Sequence

<220>
<223> amino acid sequence of mAb1 variable heavy chain region (mAb1-VH)

<400> 5

Asp Val Gln Leu Gln Glu Ser Gly Pro Gly Leu Val Lys Pro Ser Gln
1               5                   10                  15

Thr Val Ser Leu Asn Cys Thr Val Ser Gly His Ser Ile Thr Asp Asp
            20                  25                  30

Asn Tyr Tyr Trp Ser Trp Ile Arg Leu Val Ser Gly Ser Lys Leu Glu
        35                  40                  45

Trp Ile Gly Tyr Ile Thr Ser Ser Gly Asn Thr Asp Asn Asn Pro Ser
    50                  55                  60

Leu Lys Ser Arg Ile Ser Val Thr Arg Asp Ile Ser Lys Asn Gln Phe
65                  70                  75                  80

Phe Leu His Leu Ser Ser Val Thr Ala Glu Asp Ser Ala Thr Tyr Tyr
                85                  90                  95

Cys Ala Arg Asp Ser Thr Asp Gly Asp Phe Tyr Phe Glu Lys Trp Gly
            100                 105                 110

Arg Gly Thr Thr Leu Thr Val Ser Ser
        115                 120

<210> 6
<211> 330
<212> PRT
<213> Artificial Sequence

<220>
<223> amino acid sequence of mAb1 constant heavy chain region

<400> 6

Ala Lys Thr Thr Ala Pro Ser Val Tyr Pro Leu Ala Pro Val Cys Gly
1               5                   10                  15

Asp Thr Ser Gly Ser Ser Val Thr Leu Gly Cys Leu Val Lys Gly Tyr

20                  25                  30

Phe Pro Glu Pro Val Thr Leu Thr Trp Asn Ser Gly Ser Leu Ser Ser
        35                  40                  45

Gly Val His Thr Phe Pro Ala Val Leu Gln Ser Asp Leu Tyr Thr Leu
    50                  55                  60

Ser Ser Ser Val Thr Val Thr Ser Ser Thr Trp Pro Ser Gln Ser Ile
65                  70                  75                  80

Thr Cys Asn Val Ala His Pro Ala Ser Ser Thr Lys Val Asp Lys Lys
                85                  90                  95

Ile Glu Pro Arg Gly Pro Thr Ile Lys Pro Cys Pro Pro Cys Lys Cys
            100                 105                 110

Pro Ala Pro Asn Leu Leu Gly Gly Pro Ser Val Phe Ile Phe Pro Pro
        115                 120                 125

Lys Ile Lys Asp Val Leu Met Ile Ser Leu Ser Pro Ile Val Thr Cys
    130                 135                 140

Val Val Val Asp Val Ser Glu Asp Asp Pro Asp Val Gln Ile Ser Trp
145                 150                 155                 160

Phe Val Asn Asn Val Glu Val His Thr Ala Gln Thr Gln Thr His Arg
                165                 170                 175

Glu Asp Tyr Asn Ser Thr Leu Arg Val Val Ser Ala Leu Pro Ile Gln
            180                 185                 190

His Gln Asp Trp Met Ser Gly Lys Glu Phe Lys Cys Lys Val Asn Asn
        195                 200                 205

Lys Asp Leu Pro Ala Pro Ile Glu Arg Thr Ile Ser Lys Pro Lys Gly
    210                 215                 220

Ser Val Arg Ala Pro Gln Val Tyr Val Leu Pro Pro Pro Glu Glu Glu
225                 230                 235                 240

Met Thr Lys Lys Gln Val Thr Leu Thr Cys Met Val Thr Asp Phe Met
                245                 250                 255

Pro Glu Asp Ile Tyr Val Glu Trp Thr Asn Asn Gly Lys Thr Glu Leu
            260                 265                 270

Asn Tyr Lys Asn Thr Glu Pro Val Leu Asp Ser Asp Gly Ser Tyr Phe
        275                 280                 285

Met Tyr Ser Lys Leu Arg Val Glu Lys Lys Asn Trp Val Glu Arg Asn
    290                 295                 300

Ser Tyr Ser Cys Ser Val Val His Glu Gly Leu His Asn His His Thr
305                 310                 315                 320

Thr Lys Ser Phe Ser Arg Thr Pro Gly Lys
                325                 330

<210>  7
<211>  363
<212>  DNA
<213>  Artificial Sequence

<220>
<223>  nucleotide sequence of mAb1 variable heavy chain region (mAb1-VH)

<400>  7
gatgtacagc ttcaggagtc aggacctggc ctggtgaagc cttctcagac agtgtccctc      60

aactgcactg tctctggcca ctctatcact gatgataatt actattggag ctggatccgg     120

ctggtttcag gaagcaaact ggagtggata gggtacataa cctccagtgg taacactgac     180

aacaatcctt ctctcaaaag tcgcatctcc gtcacccggg acatttccaa gaaccagttc     240

ttcctgcact tgagctctgt gactgctgaa gattcagcca catattactg tgcaagagat     300

tcaactgatg gtgactttta ctttgaaaaa tggggccgag gcaccactct cacagtctcc     360

tca                                                                   363

<210>  8
<211>  990
<212>  DNA
<213>  Artificial Sequence

<220>
<223>  nucleotide sequence of mAb1 constant heavy chain region

<400>  8
gccaaaacaa cagccccatc ggtctatcca ctggcccctg tgtgtggaga tacaagtggc      60

tcctcggtga ctctaggatg cctggtcaag ggttatttcc ctgagccagt gaccttgacc     120

tggaactctg gatccctgtc cagtggtgtg cacaccttcc cagctgtcct gcagtctgac     180

ctctacaccc tcagcagctc agtgactgta acctcgagca cctggcccag ccagtccatc     240

acctgcaatg tggcccaccc ggcaagcagc accaaggtgg acaagaaaat tgagcccaga     300

gggcccacaa tcaagccctg tcctccatgc aaatgcccag cacctaacct cttgggtgga     360

ccatccgtct tcatcttccc tccaaagatc aaggatgtac tcatgatctc cctgagcccc     420

atagtcacat gtgtggtggt ggatgtgagc gaggatgacc cagatgtcca gatcagctgg 480

tttgtgaaca acgtggaagt acacacagct cagacacaaa cccatagaga ggattacaac 540

agtactctcc gggtggtcag tgccctcccc atccagcacc aggactggat gagtggcaag 600

gagttcaaat gcaaggtcaa caacaaagac ctcccagcgc ccatcgagag aaccatctca 660

aaacccaaag ggtcagtaag agctccacag gtatatgtct tgcctccacc agaagaagag 720

atgactaaga aacaggtcac tctgacctgc atggtcacag acttcatgcc tgaagacatt 780

tacgtggagt ggaccaacaa cgggaaaaca gagctaaact acaagaacac tgaaccagtc 840

ctggactctg atggttctta cttcatgtac agcaagctga gagtggaaaa gaagaactgg 900

gtggaaagaa atagctactc ctgttcagtg gtccacgagg gtctgcacaa tcaccacacg 960

actaagagct tctcccggac tccgggtaaa 990

<210> 9
<211> 108
<212> PRT
<213> Artificial Sequence

<220>
<223> amino acid sequence of 22C6 variable light chain domain (22C6-VL)

<400> 9

Glu Ile Val Leu Thr Gln Ser Pro Thr Thr Met Ala Ala Ser Pro Gly
1               5                   10                  15

Glu Lys Ile Thr Ile Thr Cys Ser Val Ser Ser Ser Ile Ser Pro Asn
            20                  25                  30

Phe Leu His Trp Tyr Gln Gln Lys Pro Gly Phe Ser Pro Lys Leu Leu
        35                  40                  45

Ile Phe Arg Thr Ser Asn Leu Ala Ser Gly Val Pro Thr Arg Phe Ser
    50                  55                  60

Gly Ser Gly Ser Gly Thr Ser Tyr Ser Leu Thr Ile Gly Thr Met Glu
65                  70                  75                  80

Ala Glu Asp Val Ala Thr Tyr Tyr Cys His Gln Gly Asn Ser Leu Pro
                85                  90                  95

Leu Thr Phe Gly Ala Gly Thr Lys Leu Glu Leu Lys
            100                 105

<210> 10
<211> 13
<212> PRT
<213> Artificial Sequence

<220>
<223>  partial amino acid sequence of 22C6 constant light chain domain
      Ck

<400>  10

Arg Ala Asp Ala Ala Pro Thr Val Ser Ile Phe Pro Pro
1               5                   10


<210>  11
<211>  324
<212>  DNA
<213>  Artificial Sequence

<220>
<223>  nucleotide sequence of 22C6 variable light chain domain (22C6-VL)

<400>  11
gaaattgttc tcacccagtc tccaaccacc atggctgcat ctcccgggga gaagatcact      60

atcacctgca gcgtcagctc aagtataagt cccaatttct tgcattggta tcagcagaag     120

ccaggattct ccccgaaact cttgattttt aggacatcca atctggcttc tggagtccca     180

actcgcttca gtggcagtgg gtctgggacc tcttactctc tcacaattgg caccatggag     240

gctgaagacg ttgccactta ttactgtcac cagggtaata gtttaccgct cacgttcggt     300

gctgggacca agctggagct gaaa                                            324


<210>  12
<211>  42
<212>  DNA
<213>  Artificial Sequence

<220>
<223>  partial nucleotide sequence of 22C6 constant light chain domain
      Ck

<400>  12
cgggctgatg ctgcaccaac tgtatccatc ttcccaccat cc                         42


<210>  13
<211>  121
<212>  PRT
<213>  Artificial Sequence

<220>
<223>  amino acid sequence of 22C6 variable heavy chain domain (22C6-VH)

<400>  13

Glu Val Gln Leu Gln Glu Ser Gly Pro Gly Leu Val Lys Pro Ser Gln
1               5                   10                  15


Thr Val Ser Leu Asn Cys Thr Val Thr Gly His Ser Ile Thr Asp Asp
            20                  25                  30

Asn Tyr Tyr Trp Ser Trp Ile Arg Leu Val Ser Gly Ser Lys Leu Glu
        35                  40                  45

Trp Ile Gly Tyr Ile Thr Ser Ala Gly Asn Thr Asp Asn Asn Pro Ser
    50                  55                  60

Leu Lys Ser Arg Ile Ser Ile Thr Arg Asp Ile Ser Lys Asn Gln Phe
65                  70                  75                  80

Phe Leu His Leu Ser Ser Val Thr Ala Glu Asp Thr Ala Thr Tyr Tyr
                85                  90                  95

Cys Ala Arg Asp Ser Thr Asp Gly Asp Phe Tyr Phe Glu Lys Trp Gly
            100                 105                 110

Gln Gly Thr Thr Leu Thr Val Ser Ser
        115                 120

<210>  14
<211>  30
<212>  PRT
<213>  Artificial Sequence

<220>
<223>  partial amino acid sequence of 22C6 constant heavy chain domain
       CH1

<400>  14

Ala Lys Thr Thr Ala Pro Ser Val Tyr Pro Leu Ala Pro Val Cys Gly
1               5                   10                  15

Asp Thr Ser Gly Ser Ser Val Thr Leu Gly Cys Leu Val Lys
            20                  25                  30

<210>  15
<211>  363
<212>  DNA
<213>  Artificial Sequence

<220>
<223>  nucleotide sequence of 22C6 variable heavy chain domain (22C6-VH)

<400>  15
gaggtgcagc tgcaggagtc aggacctggc ctggtgaagc cttctcagac agtgtccctc      60

aactgcactg tcactggcca ctctatcact gatgataatt actattggag ctggatccgg     120

ctggtttcag gaagcaaact ggagtggata gggtacataa cctccgctgg taacactgac     180

aacaatcctt ctctcaaaag tcgcatctcc atcacccggg acatttccaa gaaccaattc     240

ttcctgcact tgagctctgt gactgctgaa gatacagcca catattactg tgcaagagat     300

tcaactgatg gtgactttta ctttgaaaaa tggggccaag gcaccactct cacagtctcc 360

tca 363

<210> 16
<211> 90
<212> DNA
<213> Artificial Sequence

<220>
<223> partial nucleotide sequence of constant 22C6 heavy chain domain
      CH1

<400> 16
gccaaaacaa cagccccatc ggtctatcca ctggcccctg tgtgtggaga tacaagtggc 60

tcctcggtga ctctaggatg cctggtcaag 90

<210> 17
<211> 654
<212> DNA
<213> Artificial Sequence

<220>
<223> full nucleotide sequence of mAb1 light chain (mAb1-LC)

<400> 17
gacattgtgc tgacccaatc tccagcttct ttggctgtgt ctctagggca gagggccacc 60

atctcctgca gagccagcga aagtgttgat aattatggca ttagttttat gcactggtac 120

cagcagaaac cgggacagcc acccaaactc ctcatctatc gtgcatccaa cctagaatct 180

gggatccctg ccaggttcag tggcagtggg tctaggacag acttcaccct caccattaat 240

cctgtggaga ctgatgatgt tgcaacctat tactgtcagc aaagtagtag ggatctgttc 300

acgttcggag gggggaccaa gctggaaata aaacgggctg atgctgcacc aactgtatcc 360

atcttcccac catccagtga gcagttaaca tctggaggtg cctcagtcgt gtgcttcttg 420

aacaacttct accccagaga catcaatgtc aagtggaaga ttgatggcag tgaacgacaa 480

aatggtgtcc tgaacagttg gactgatcag gacagcaaag acagcaccta cagcatgagc 540

agcaccctca cattgaccaa ggacgagtat gaacgacata acagctatac ctgtgaggcc 600

actcacaaga catcaacttc acccatcgtc aagagcttca acaggaatga gtgt 654

<210> 18
<211> 218
<212> PRT
<213> Artificial Sequence

<220>
<223> full amino acid sequence of mAb1 light chain (mAb1-LC)

<400> 18

Asp Ile Val Leu Thr Gln Ser Pro Ala Ser Leu Ala Val Ser Leu Gly

1               5                   10                  15

Gln Arg Ala Thr Ile Ser Cys Arg Ala Ser Glu Ser Val Asp Asn Tyr
            20                  25                  30

Gly Ile Ser Phe Met His Trp Tyr Gln Gln Lys Pro Gly Gln Pro Pro
        35                  40                  45

Lys Leu Leu Ile Tyr Arg Ala Ser Asn Leu Glu Ser Gly Ile Pro Ala
    50                  55                  60

Arg Phe Ser Gly Ser Gly Ser Arg Thr Asp Phe Thr Leu Thr Ile Asn
65                  70                  75                  80

Pro Val Glu Thr Asp Asp Val Ala Thr Tyr Tyr Cys Gln Gln Ser Ser
                85                  90                  95

Arg Asp Leu Phe Thr Phe Gly Gly Gly Thr Lys Leu Glu Ile Lys Arg
            100                 105                 110

Ala Asp Ala Ala Pro Thr Val Ser Ile Phe Pro Pro Ser Ser Glu Gln
        115                 120                 125

Leu Thr Ser Gly Gly Ala Ser Val Val Cys Phe Leu Asn Asn Phe Tyr
    130                 135                 140

Pro Arg Asp Ile Asn Val Lys Trp Lys Ile Asp Gly Ser Glu Arg Gln
145                 150                 155                 160

Asn Gly Val Leu Asn Ser Trp Thr Asp Gln Asp Ser Lys Asp Ser Thr
                165                 170                 175

Tyr Ser Met Ser Ser Thr Leu Thr Leu Thr Lys Asp Glu Tyr Glu Arg
            180                 185                 190

His Asn Ser Tyr Thr Cys Glu Ala Thr His Lys Thr Ser Thr Ser Pro
        195                 200                 205

Ile Val Lys Ser Phe Asn Arg Asn Glu Cys
    210                 215

<210>  19
<211>  1353
<212>  DNA
<213>  Artificial Sequence

<220>
<223>  full nucleotide sequence of mAb1 heavy chain (mAb1-HC)

<400>  19
gatgtacagc ttcaggagtc aggacctggc ctggtgaagc cttctcagac agtgtccctc      60

aactgcactg tctctggcca ctctatcact gatgataatt actattggag ctggatccgg     120

ctggtttcag gaagcaaact ggagtggata gggtacataa cctccagtgg taacactgac     180

aacaatcctt ctctcaaaag tcgcatctcc gtcacccggg acatttccaa gaaccagttc     240

ttcctgcact tgagctctgt gactgctgaa gattcagcca catattactg tgcaagagat     300

tcaactgatg gtgactttta ctttgaaaaa tggggccgag gcaccactct cacagtctcc     360

tcagccaaaa caacagcccc atcggtctat ccactggccc ctgtgtgtgg agatacaagt     420

ggctcctcgg tgactctagg atgcctggtc aagggttatt tccctgagcc agtgaccttg     480

acctggaact ctggatccct gtccagtggt gtgcacacct tcccagctgt cctgcagtct     540

gacctctaca ccctcagcag ctcagtgact gtaacctcga gcacctggcc cagccagtcc     600

atcacctgca atgtggccca cccggcaagc agcaccaagg tggacaagaa aattgagccc     660

agagggccca caatcaagcc ctgtcctcca tgcaaatgcc cagcacctaa cctcttgggt     720

ggaccatccg tcttcatctt ccctccaaag atcaaggatg tactcatgat ctccctgagc     780

cccatagtca catgtgtggt ggtggatgtg agcgaggatg acccagatgt ccagatcagc     840

tggtttgtga acaacgtgga agtacacaca gctcagacac aaacccatag agaggattac     900

aacagtactc tccgggtggt cagtgccctc cccatccagc accaggactg gatgagtggc     960

aaggagttca aatgcaaggt caacaacaaa gacctcccag cgcccatcga gagaaccatc    1020

tcaaaaccca aagggtcagt aagagctcca caggtatatg tcttgcctcc accagaagaa    1080

gagatgacta agaaacaggt cactctgacc tgcatggtca cagacttcat gcctgaagac    1140

atttacgtgg agtggaccaa caacgggaaa acagagctaa actacaagaa cactgaacca    1200

gtcctggact ctgatggttc ttacttcatg tacagcaagc tgagagtgga aaagaagaac    1260

tgggtggaaa gaaatagcta ctcctgttca gtggtccacg agggtctgca caatcaccac    1320

acgactaaga gcttctcccg gactccgggt aaa                                 1353

<210>  20
<211>  451
<212>  PRT
<213>  Artificial Sequence

<220>
<223>  full amino acid sequence of mAb1 heavy chain (mAb1-HC)

<400>  20

Asp Val Gln Leu Gln Glu Ser Gly Pro Gly Leu Val Lys Pro Ser Gln
1               5                   10                  15

Thr Val Ser Leu Asn Cys Thr Val Ser Gly His Ser Ile Thr Asp Asp

            20                  25                  30

Asn Tyr Tyr Trp Ser Trp Ile Arg Leu Val Ser Gly Ser Lys Leu Glu
        35                  40                  45

Trp Ile Gly Tyr Ile Thr Ser Ser Gly Asn Thr Asp Asn Asn Pro Ser
    50                  55                  60

Leu Lys Ser Arg Ile Ser Val Thr Arg Asp Ile Ser Lys Asn Gln Phe
65                  70                  75                  80

Phe Leu His Leu Ser Ser Val Thr Ala Glu Asp Ser Ala Thr Tyr Tyr
                85                  90                  95

Cys Ala Arg Asp Ser Thr Asp Gly Asp Phe Tyr Phe Glu Lys Trp Gly
            100                 105                 110

Arg Gly Thr Thr Leu Thr Val Ser Ser Ala Lys Thr Thr Ala Pro Ser
        115                 120                 125

Val Tyr Pro Leu Ala Pro Val Cys Gly Asp Thr Ser Gly Ser Ser Val
    130                 135                 140

Thr Leu Gly Cys Leu Val Lys Gly Tyr Phe Pro Glu Pro Val Thr Leu
145                 150                 155                 160

Thr Trp Asn Ser Gly Ser Leu Ser Ser Gly Val His Thr Phe Pro Ala
                165                 170                 175

Val Leu Gln Ser Asp Leu Tyr Thr Leu Ser Ser Ser Val Thr Val Thr
            180                 185                 190

Ser Ser Thr Trp Pro Ser Gln Ser Ile Thr Cys Asn Val Ala His Pro
        195                 200                 205

Ala Ser Ser Thr Lys Val Asp Lys Lys Ile Glu Pro Arg Gly Pro Thr
    210                 215                 220

Ile Lys Pro Cys Pro Pro Cys Lys Cys Pro Ala Pro Asn Leu Leu Gly
225                 230                 235                 240

Gly Pro Ser Val Phe Ile Phe Pro Pro Lys Ile Lys Asp Val Leu Met
                245                 250                 255

Ile Ser Leu Ser Pro Ile Val Thr Cys Val Val Val Asp Val Ser Glu
            260                 265                 270

Asp Asp Pro Asp Val Gln Ile Ser Trp Phe Val Asn Asn Val Glu Val
        275                 280                 285

His Thr Ala Gln Thr Gln Thr His Arg Glu Asp Tyr Asn Ser Thr Leu
    290                 295                 300

Arg Val Val Ser Ala Leu Pro Ile Gln His Gln Asp Trp Met Ser Gly
305                 310                 315                 320

Lys Glu Phe Lys Cys Lys Val Asn Asn Lys Asp Leu Pro Ala Pro Ile
                325                 330                 335

Glu Arg Thr Ile Ser Lys Pro Lys Gly Ser Val Arg Ala Pro Gln Val
            340                 345                 350

Tyr Val Leu Pro Pro Pro Glu Glu Glu Met Thr Lys Lys Gln Val Thr
        355                 360                 365

Leu Thr Cys Met Val Thr Asp Phe Met Pro Glu Asp Ile Tyr Val Glu
    370                 375                 380

Trp Thr Asn Asn Gly Lys Thr Glu Leu Asn Tyr Lys Asn Thr Glu Pro
385                 390                 395                 400

Val Leu Asp Ser Asp Gly Ser Tyr Phe Met Tyr Ser Lys Leu Arg Val
                405                 410                 415

Glu Lys Lys Asn Trp Val Glu Arg Asn Ser Tyr Ser Cys Ser Val Val
            420                 425                 430

His Glu Gly Leu His Asn His His Thr Thr Lys Ser Phe Ser Arg Thr
        435                 440                 445

Pro Gly Lys
    450

<210>  21
<211>  10
<212>  PRT
<213>  Artificial Sequence

<220>
<223>  amino acid sequence of CDR1 of mAb1-VL

<400>  21

Glu Ser Val Asp Asn Tyr Gly Ile Ser Phe
1               5                   10

<210>  22

<211>  3
<212>  PRT
<213>  Artificial Sequence

<220>
<223>  amino acid sequence of CDR2 of mAb1-VL

<400>  22

Arg Ala Ser
1


<210>  23
<211>  9
<212>  PRT
<213>  Artificial Sequence

<220>
<223>  amino acid sequence of CDR3 of mAb1-VL

<400>  23

Gln Gln Ser Ser Arg Asp Leu Phe Thr
1               5


<210>  24
<211>  10
<212>  PRT
<213>  Artificial Sequence

<220>
<223>  amino acid sequence of CDR1 of mAb1-VH

<400>  24

Gly His Ser Ile Thr Asp Asp Asn Tyr Tyr
1               5                   10


<210>  25
<211>  7
<212>  PRT
<213>  Artificial Sequence

<220>
<223>  amino acid sequence of CDR2 of mAb1-VH

<400>  25

Ile Thr Ser Ser Gly Asn Thr
1               5


<210>  26
<211>  13
<212>  PRT
<213>  Artificial Sequence

<220>
<223>  amino acid sequence of CDR3 of mAb1-VH

<400>  26

Ala Arg Asp Ser Thr Asp Gly Asp Phe Tyr Phe Glu Lys
1               5                   10


<210>  27
<211>  7
<212>  PRT
<213>  Artificial Sequence

<220>
<223>  amino acid sequence of CDR1 of 22C6-VL

<400>  27

Ser Ser Ile Ser Pro Asn Phe
1               5


<210>  28
<211>  3
<212>  PRT
<213>  Artificial Sequence

<220>
<223>  amino acid sequence of CDR2 of 22C6-VL

<400>  28

Arg Thr Ser
1


<210>  29
<211>  9
<212>  PRT
<213>  Artificial Sequence

<220>
<223>  amino acid sequence of CDR3 of 22C6-VL

<400>  29

His Gln Gly Asn Ser Leu Pro Leu Thr
1               5


<210>  30
<211>  10
<212>  PRT
<213>  Artificial Sequence

<220>
<223>  amino acid sequence of CDR1 of 22C6-VH

<400>  30

Gly His Ser Ile Thr Asp Asp Asn Tyr Tyr
1               5                   10


<210>  31

<211>  7
<212>  PRT
<213>  Artificial Sequence

<220>
<223>  amino acid sequence of CDR2 of 22C6-VH

<400>  31

Ile Thr Ser Ala Gly Asn Thr
1               5


<210>  32
<211>  13
<212>  PRT
<213>  Artificial Sequence

<220>
<223>  amino acid sequence of CDR3 of 22C6-VH

<400>  32

Ala Arg Asp Ser Thr Asp Gly Asp Phe Tyr Phe Glu Lys
1               5                   10


<210>  33
<211>  97
<212>  PRT
<213>  Artificial Sequence

<220>
<223>  amino acid sequence of constant CH1 domain of mAb1-VH

<400>  33

Ala Lys Thr Thr Ala Pro Ser Val Tyr Pro Leu Ala Pro Val Cys Gly
1               5                   10                  15


Asp Thr Ser Gly Ser Ser Val Thr Leu Gly Cys Leu Val Lys Gly Tyr
            20                  25                  30


Phe Pro Glu Pro Val Thr Leu Thr Trp Asn Ser Gly Ser Leu Ser Ser
        35                  40                  45


Gly Val His Thr Phe Pro Ala Val Leu Gln Ser Asp Leu Tyr Thr Leu
    50                  55                  60


Ser Ser Ser Val Thr Val Thr Ser Ser Thr Trp Pro Ser Gln Ser Ile
65                  70                  75                  80


Thr Cys Asn Val Ala His Pro Ala Ser Ser Thr Lys Val Asp Lys Lys
                85                  90                  95


Ile

<210>  34
<211>  110
<212>  PRT
<213>  Artificial Sequence

<220>
<223>  amino acid sequence of constant CH2 domain of mAb1-VH

<400>  34

Ala Pro Asn Leu Leu Gly Gly Pro Ser Val Phe Ile Phe Pro Pro Lys
1               5                   10                  15

Ile Lys Asp Val Leu Met Ile Ser Leu Ser Pro Ile Val Thr Cys Val
            20                  25                  30

Val Val Asp Val Ser Glu Asp Asp Pro Asp Val Gln Ile Ser Trp Phe
        35                  40                  45

Val Asn Asn Val Glu Val His Thr Ala Gln Thr Gln Thr His Arg Glu
    50                  55                  60

Asp Tyr Asn Ser Thr Leu Arg Val Val Ser Ala Leu Pro Ile Gln His
65                  70                  75                  80

Gln Asp Trp Met Ser Gly Lys Glu Phe Lys Cys Lys Val Asn Asn Lys
                85                  90                  95

Asp Leu Pro Ala Pro Ile Glu Arg Thr Ile Ser Lys Pro Lys
            100                 105                 110


<210>  35
<211>  107
<212>  PRT
<213>  Artificial Sequence

<220>
<223>  amino acid sequence of constant CH3 domain of mAb1-VH

<400>  35

Gly Ser Val Arg Ala Pro Gln Val Tyr Val Leu Pro Pro Pro Glu Glu
1               5                   10                  15

Glu Met Thr Lys Lys Gln Val Thr Leu Thr Cys Met Val Thr Asp Phe
            20                  25                  30

Met Pro Glu Asp Ile Tyr Val Glu Trp Thr Asn Asn Gly Lys Thr Glu
        35                  40                  45

Leu Asn Tyr Lys Asn Thr Glu Pro Val Leu Asp Ser Asp Gly Ser Tyr

50                  55                  60

Phe Met Tyr Ser Lys Leu Arg Val Glu Lys Lys Asn Trp Val Glu Arg
65                  70                  75                  80

Asn Ser Tyr Ser Cys Ser Val Val His Glu Gly Leu His Asn His His
                85                  90                  95

Thr Thr Lys Ser Phe Ser Arg Thr Pro Gly Lys
            100                 105

<210>  36
<211>  16
<212>  PRT
<213>  Artificial Sequence

<220>
<223>  amino acid sequence of hinge region between CH1 and CH2 of
       mAb1-VH

<400>  36

Glu Pro Arg Gly Pro Thr Ile Lys Pro Cys Pro Pro Cys Lys Cys Pro
1               5                   10                  15

<210>  37
<211>  148
<212>  PRT
<213>  Homo sapiens

<400>  37

Met Lys Leu Leu Val Leu Ala Val Leu Leu Thr Val Ala Ala Ala Asp
1               5                   10                  15

Ser Gly Ile Ser Pro Arg Ala Val Trp Gln Phe Arg Lys Met Ile Lys
            20                  25                  30

Cys Val Ile Pro Gly Ser Asp Pro Phe Leu Glu Tyr Asn Asn Tyr Gly
        35                  40                  45

Cys Tyr Cys Gly Leu Gly Gly Ser Gly Thr Pro Val Asp Glu Leu Asp
    50                  55                  60

Lys Cys Cys Gln Thr His Asp Asn Cys Tyr Asp Gln Ala Lys Lys Leu
65                  70                  75                  80

Asp Ser Cys Lys Phe Leu Leu Asp Asn Pro Tyr Thr His Thr Tyr Ser
                85                  90                  95

Tyr Ser Cys Ser Gly Ser Ala Ile Thr Cys Ser Ser Lys Asn Lys Glu
            100                 105                 110

Cys Glu Ala Phe Ile Cys Asn Cys Asp Arg Asn Ala Ala Ile Cys Phe
        115                 120                 125

Ser Lys Ala Pro Tyr Asn Lys Ala His Lys Asn Leu Asp Thr Lys Lys
    130                 135                 140

Tyr Cys Gln Ser
145

<210>  38
<211>  148
<212>  PRT
<213>  Macaque

<400>  38

Met Lys Leu Leu Val Leu Ala Val Leu Leu Thr Val Ala Ala Ala Glu
1               5                   10                  15

Ser Gly Ile Ser Ser Arg Ala Val Trp Gln Phe Arg Lys Leu Ile Lys
            20                  25                  30

Cys Val Ile Pro Gly Ser Asp Pro Tyr Leu Glu Tyr Asn Asn Tyr Gly
        35                  40                  45

Cys Tyr Cys Gly Leu Gly Gly Ser Gly Thr Pro Val Asp Glu Leu Asp
    50                  55                  60

Lys Cys Cys Gln Thr His Asp Asn Cys Tyr Asp Gln Ala Lys Lys Leu
65                  70                  75                  80

Ser Ser Cys Lys Phe Leu Leu Asp Asn Pro Tyr Thr His Thr Tyr Ser
                85                  90                  95

Tyr Ser Cys Ser Asn Ser Glu Ile Thr Cys Ser Ser Lys Asn Lys Glu
            100                 105                 110

Cys Glu Ala Phe Ile Cys Asn Cys Asp Arg Asn Ala Ala Ile Cys Phe
        115                 120                 125

Ser Lys Ala Pro Tyr Asn Lys Glu His Lys Asn Leu Asp Ser Lys Lys
    130                 135                 140

Tyr Cys Gln Ser
145

## Description

The present invention relates to anti-PLA2G1B monoclonal antibodies and the uses thereof. The invention more particularly discloses new monoclonal antibodies which can bind and inhibit human or simian PLA2G1B. The invention is particularly useful for detecting, monitoring or treating an immunodeficiency or an associated disease caused, for example, by HIV, SIV or SHIV.

### BACKGROUND OF THE INVENTION

The group 1B pancreatic phospholipase A2 (PLA2G1B) is a low molecular weight (14 kDa), highly stable (7 disulfide bonds), secreted protein, that catalyzes the hydrolysis of the sn-2 fatty acyl bond of phospholipids to release free fatty acids and lysophospholipids (Lambeau & Gelb 2008). PLA2G1B is first secreted in the pancreatic juice as an inactive pro form (pro-sPLA2G1B), which is activated by proteolytic cleavage of a propeptide, leading to the active mature secreted form (sPLA2G1B). *In vitro* experiments suggested that trypsin and plasmin are able to activate pro-sPLA2G1B with good potency (Nakano et al. 1994). In addition to a role in the gastrointestinal tract, PLA2G1B is also circulating in human plasma (Nishijima et al. 1983, Eskola et al. 1983, Sternby, 1984, Kitagawa et al. 1991).

The inventors have previously characterized sPLA2G1B as a key endogenous factor of the immune response, and they demonstrated that sPLA2G1B plays a crucial role in the mechanism underlying the unresponsiveness of CD4 T lymphocytes observed for example in viremic HIV-infected subjects (WO2015/097140). The inventors have also proposed and documented that PLA2G1B modulators are effective for treating disorders associated with an immune deficiency.

SIV infection in non-human primates so far remains the best model for the study of HIV transmission, pathogenesis and control (Barre-Sinoussi F et al., 2013). In 1998, studies in rhesus macaques showed that, during the acute phase, within just a few days of SIV infection, there is significant CD4 T cell depletion in the gastrointestinal tract (Veazey, R.S et al. 1998). Gut mucosa studies of SIV-infected macaques and HIV-infected humans have revealed virus-induced changes of intestinal CD4, CD8 T cells, innate lymphoid cells, myeloid cells, and of the local cytokine/chemokine network in addition to epithelial injuries (Ponte R et al. 2016). SHIV, a virus combining parts of the HIV and SIV genomes, was created for various research purposes, including analyzing how different parts of the virus respond to different antimicrobial drugs and vaccines. SHIV infection resembles HIV-1 infection and causes irreversible depletions of CD4 T cells in infected monkeys leading to symptomatic immunodeficiency associated with opportunistic infections and a fatal clinical outcome in untreated monkeys (Nishimura Y et al. 2017). The comparison between pathogenic and non-pathogenic simian models was crucial in determining that viral replication is not the only factor involved in disease progression (Rey-Cuillé MA et al. 1998; Müller-Trutwin MC et al., 1996; Sodora DL et al. 2009).

The role of macaque PLA2G1B in the immunodeficiency observed in SIV- and SHIV-infected monkeys has not yet been investigated at all.

There is a need for methods allowing an accurate and sensitive detection of PLA2G1B in various mammalian species. There is also a need for new treatments of diseases caused by HIV, SIV or SHIV.

### SUMMARY OF THE INVENTION

The present invention provides therapeutic antibodies against mammalian PLA2G1B that can bind human or simian PLA2G1B protein and inhibit an enzymatic activity of PLA2G1B.

A first object of the present invention relates to a monoclonal antibody (mAb) or an antigen-binding fragment thereof, wherein said antibody or fragment thereof comprises a light chain variable region comprising (i) a CDR1 comprising, consisting, or consisting essentially of SEQ ID NO: 21, and/or (ii) a CDR2 comprising, consisting, or consisting essentially of SEQ ID NO: 22, and/or (iii) a CDR3 comprising, consisting, or consisting essentially of SEQ ID NO: 23.

Another object of the present invention relates to a monoclonal antibody or an antigen-binding fragment thereof, wherein said antibody or fragment thereof comprises a heavy chain variable region comprising (i) a CDR1 comprising, consisting, or consisting essentially of SEQ ID NO: 24, and/or (ii) a CDR2 comprising, consisting, or consisting essentially of SEQ ID NO: 25, and/or (iii) a CDR3 comprising, consisting, or consisting essentially of SEQ ID NO: 26.

Another object of the invention relates to a monoclonal comprising a light chain variable region comprising SEQ ID NO: 1 and/or a heavy chain variable region comprising SEQ ID NO: 5.

Another object of the invention relates to a monoclonal antibody comprising a light chain sequence of SEQ ID NO: 18, and/or a heavy chain sequence of SEQ ID NO: 20.

A further object of the invention relates to a nucleic acid encoding an antibody or an antigen-binding fragment thereof, as described herein, or a light or heavy chain thereof, or a variable domain thereof. The invention also relates to a vector comprising a nucleic acid as described herein, as well as to a recombinant host cell containing such a vector or such a nucleic acid.

A further object of the invention is a composition comprising (i) an antibody or an-antigen-binding fragment thereof, a nucleic acid, a vector, or a recombinant host cell, as described herein, and (ii) a pharmaceutically acceptable carrier or excipient.

The invention also relates to an antibody or an antigen-binding fragment thereof, a nucleic acid, a vector, a recombinant host cell, or a composition, as described herein, for use as a medicament, particularly in the treatment of an immunodeficiency or an associated disease. A further object of the invention relates to a method for producing an antibody as defined herein, comprising culturing a cell as defined herein under conditions suitable for expression of the antibody, and optionally recovering said antibody.

A further object of the invention relates to a kit comprising a least one antibody or an antigen-binding fragment thereof, as described herein, and a reagent to perform, detect or quantify an immune reaction, or an antibody-antigen complex.

A further object of the invention relates to a method for detecting or quantifying mammalian PLA2G1B protein in a sample using an antibody or an antigen-binding fragment thereof as defined herein. The methods are particularly useful for detecting or monitoring pathological conditions in subjects that are associated with an activity of PLA2G1B.

The invention also relates to a method for treating a subject in need thereof, particularly a mammalian subject having an immunodeficiency or an associated disease, comprising administering to the subject an antibody or an-antigen-binding fragment thereof, a nucleic acid, a vector, a recombinant host cell, or a composition, as defined herein.

The therapeutic antibodies of the invention may be used in any mammal, particularly any human or simian subject in need thereof. They are suitable to inhibit PLA2G1B and correct any disease associated with undesirable activity of said protein.

### LEGEND TO THE FIGURES

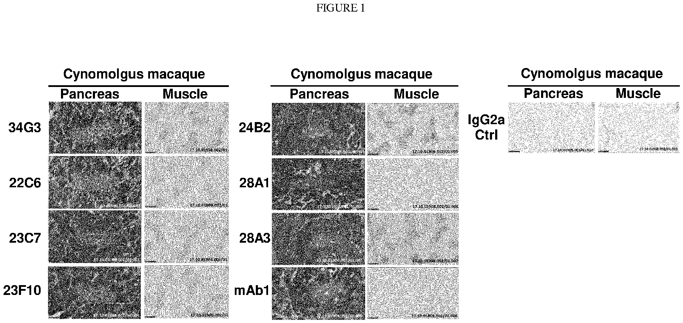

**Figure 1****:** Identification of anti-PLA2G1B monoclonal antibodies (mAbs) that inhibit human PLA2G1B enzymatic activity and label cynomolgus macaque tissues. Cynomolgus macaque pancreas was used as positive tissue for active cynomolgus macaque expression and cynomolgus macaque muscle was used as negative tissue for PLA2G1B expression. Frozen pancreas and muscle sections were labelled with 20 µg/ml of 22C6, mAb1, 34G3, 23C7, 23F10, 24B2, 28A1, 28A3 antibodies and IgG2a control isotype antibodies. Scale bar is 100 µm.

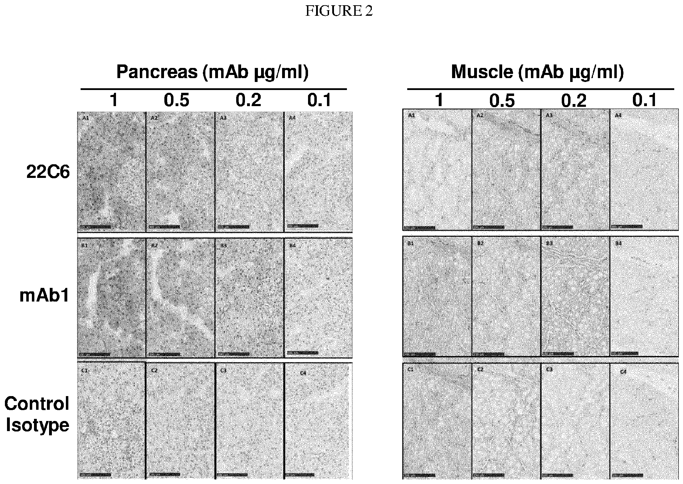

**Figure 2****:** 22C6 and mAb1 preferentially label exocrine part of cynomolgus macaque pancreas. Frozen cynomolgus macaque pancreas and muscle sections were labelled with several concentrations of 22C6 or mAb1 antibody or IgG2a control isotype (0.1, 0.2, 0.5 and 1 µg/ml). Scale bar is 100 µm.

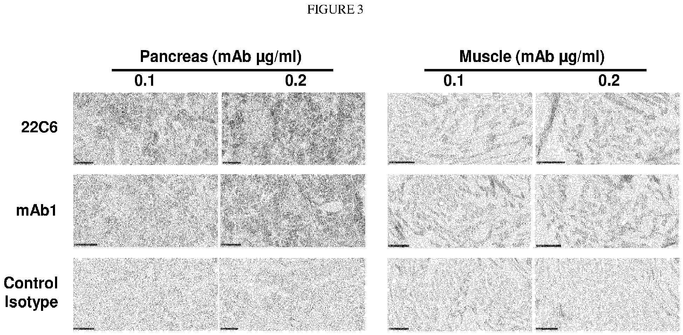

**Figure 3****:** 22C6 and mAb1 preferentially label exocrine part of human pancreas. Frozen human pancreas and muscle sections were labelled with 0.1 and 0.2 µg/ml of 22C6 or mAb1 antibody or IgG2a control isotype. Scale bar is 100 µm.

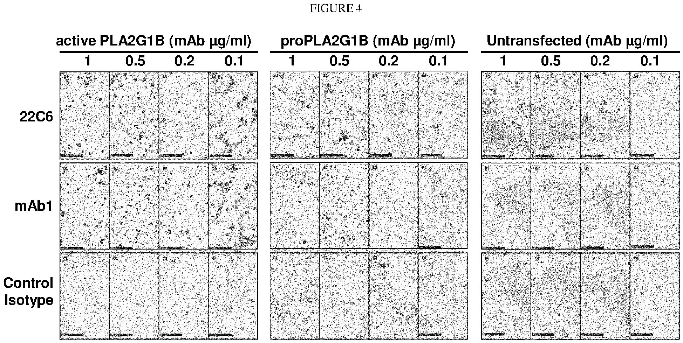

**Figure 4****:** 22C6 and mAb1 preferentially label active PLA2G1B in CHO cells expressing human active PLA2G1B, proPLA2G1B or untransfected. Frozen CHO cells transfected to overexpress human active PLA2G1B (active PLA2G1B), human proPLA2G1B (proPLA2G1B) or untransfected were labelled with several concentrations of 22C6 or mAb1 antibody or IgG2a control isotype (0.1, 0.2, 0.5 and 1 µg/ml) to determine the labelling condition specific of active PLA2G1B. Scale bar is 100 µm.

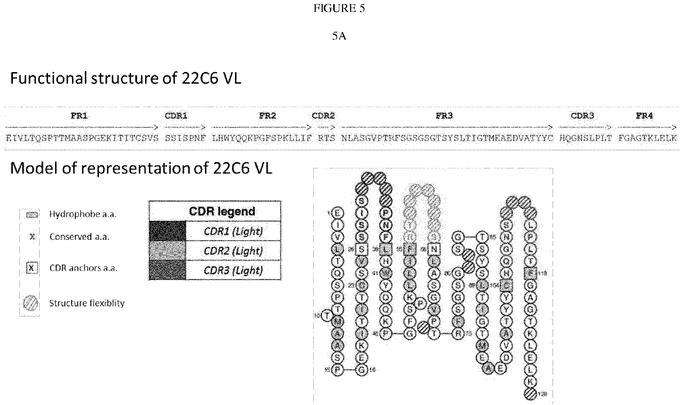

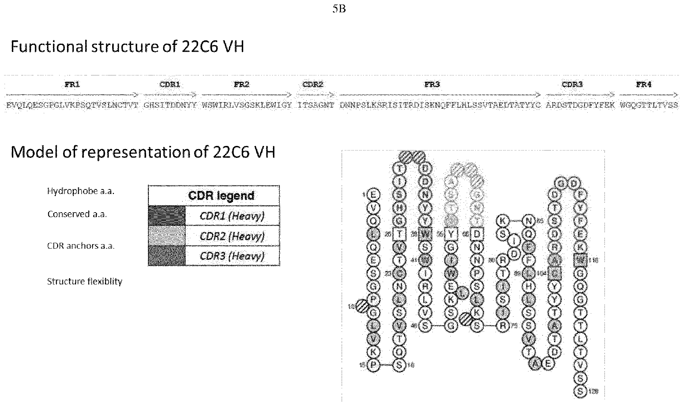

**Figure 5****:** Sequences of 22C6 light (VL) and heavy (VH) chain variable regions. 22C6 is the abbreviation of 22C6-3A6 clone. **(A)** Functional structure and model of representation (IMGT 'Colliers de Perles' according to Pommié et al., 2004) of 22C6 VL. 22C6 VL is a rearranged and productive IGK sequence, Kappa isotype, with no equivalent in database (Uniprot databank-UniprotKB). (**B**) Functional structure and model of representation (IMGT Colliers de Perles) of 22C6 VH. 22C6 VH is a rearranged and productive IGH sequence, IgG2a isotype, with no equivalent in database (Uniprot databank-UniprotKB).

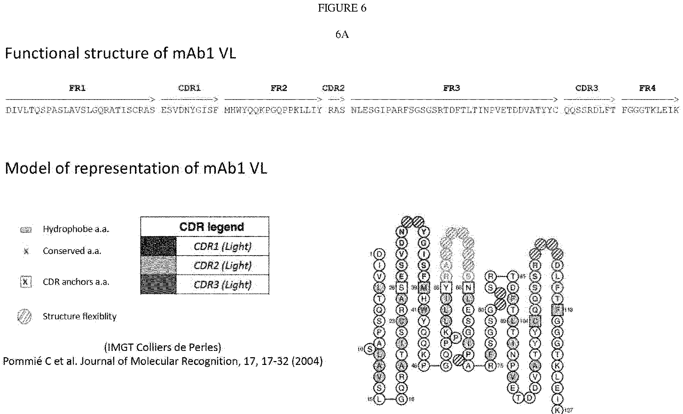

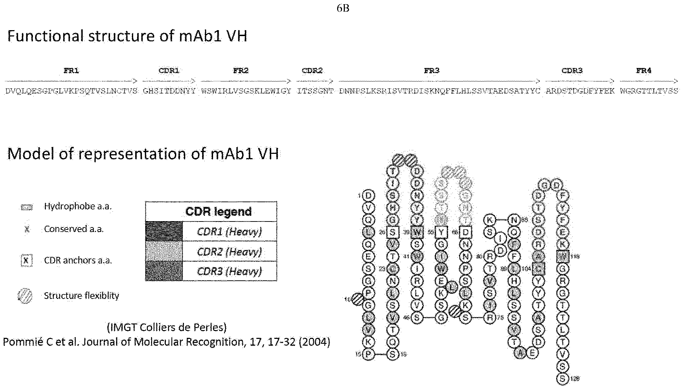

**Figure** 6: Sequences of mAb1 light (VL) and heavy (VH) chain variable regions. (**A**) Functional structure and model of representation (IMGT Colliers de Perles) of mAb1 VL. mAb 1 VL is a rearranged and productive IGK sequence, Kappa isotype, with no equivalent in database (Uniprot databank-UniprotKB). (**B**) Functional structure and model of representation (IMGT Colliers de Perles) of mAb1 VH. mAb1 VH is a rearranged and productive IGH sequence, IgG2a isotype, with no equivalent in database (Uniprot databank-UniprotKB).

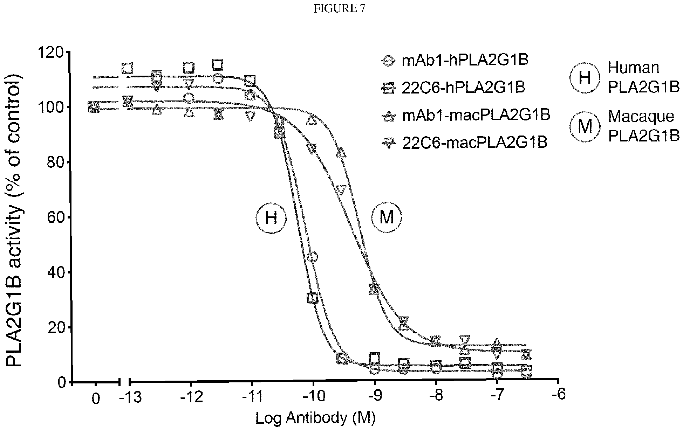

**Figure 7****:** 22C6 and mAb1 antibodies inhibit human and cynomolgus macaque PLA2G1B enzymatic activity. Human (hPLA2G1B, H) and cynomolgus macaque (macPLA2G1B, M) PLA2G1B were incubated with several concentrations of 22C6 or mAb1 antibody. Then PLA2G1B enzymatic activity was measured on *E. Coli* membranes previously labelled with tritiated oleic acid. Results are presented as percentage of activity of PLA2G1B incubated with control isotype. Data were fitted with a 4PL logistic non-linear regression model and the relative IC50 values were estimated from the model.

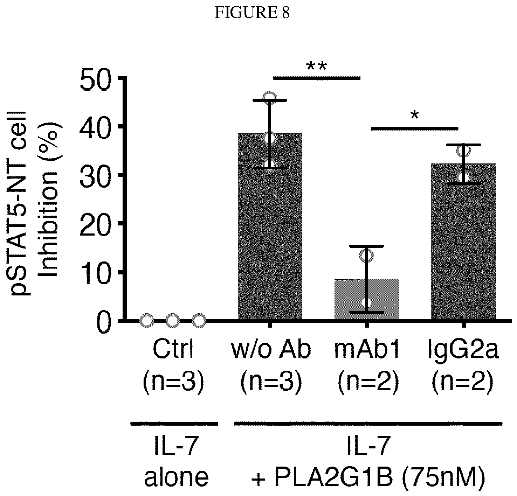

**Figure 8****:** mAb1 antibody strongly blocks PLA2G1B-dependent inhibition of pSTAT5 NT in primary human CD4 T cells. PLA2G1B was pretreated or not with an anti-PLA2G1B antibody (mAb1, 100µg/ml) or its control isotype (IgG2a, 100µg/ml). Healthy donor CD4 T cells (n=3 or 2 donors as indicated) were incubated or not with antibodies-pretreated PLA2G1B (75 nM) and then stimulated with IL-7 (2 nM). Results show the mean ± SD of percentage of inhibition of pSTAT5-NT cell response to IL-7 normalized to IL-7 response in absence of PLA2G1B (Ctrl) in two to three independent experiments (1 donor/experiment). *p<0.05 and **p<0.01 by ANOVA with Tukey's correction for multiple comparisons.

### DETAILED DESCRIPTION OF THE INVENTION

The present invention relates to novel antibodies and uses thereof. The invention relates, in particular, to anti-PLA2G1B antibodies that bind human and/or simian PLA2G1B. The invention also provides methods for detecting or dosing PLA2G1B in a sample using such antibodies or fragments thereof, as well as methods for inhibiting said enzyme in a mammal using said antibodies and fragments.

Inventors have previously shown that PLA2G1B activity induces anergy and lymphopenia of human CD4 T cells in HIV-infected patients (Pothlichet et al., 2020). In the present application, the inventors have identified antibodies able to specifically bind to human and simian active PLA2G1B. The inventors have also found that these antibodies effectively inhibit the enzymatic activity of human and simian PLA2G1B. Furthermore, the inventors have also demonstrated that the anti-PLA2G1B antibodies of the invention strongly block PLA2G1B inhibitory activity on CD4 T cells. These antibodies thus exhibit remarkable properties for therapeutic and diagnostic uses, in human and veterinary fields.

### Definitions

As used herein, the term "PLA2G1B" designates group IB pancreatic phospholipase A2. PLA2G1B has been identified and cloned from various mammalian species. The human PLA2G1B protein is disclosed, for instance, in Lambeau and Gelb, 2008. The sequence is available on Genbank No. NP_000919. The amino acid sequence of an exemplary human PLA2G1B is shown below (SEQ ID NO: 37).

MKLLVLAVLL TVAAADSGIS PRAVWQFRKM IKCVIPGSDP FLEYNNYGCY
CGLGGSGTPV DELDKCCQTH DNCYDQAKKL DSCKFLLDNP YTHTYSYSCS
GSAITCSSKN KECEAFICNC DRNAAICFSK APYNKAHKNL DTKKYCQS

Amino acids 1 to 15 of SEQ ID NO: 37 (underlined) are a signal sequence, and amino acids 16 to 22 of SEQ ID NO: 37 (in bold) are a propeptide sequence.

The macaque PLA2G1B protein sequence is available on Genbank No. XP_005572452.1. The amino acid sequence of an exemplary macaque PLA2G1B is shown below (SEQ ID NO: 38).

MKLLVLAVLL TVAAAESGIS SRAVWQFRKL IKCVIPGSDP YLEYNNYGCY
CGLGGSGTPV DELDKCCQTH DNCYDQAKKL SSCKFLLDNP YTHTYSYSCS
NSEITCSSKN KECEAFICNC DRNAAICFSK APYNKEHKNL DSKKYCQS

Amino acids 1 to 15 of SEQ ID NO: 38 (underlined) are a signal sequence, and amino acids 16 to 22 of SEQ ID NO: 38 (in bold) are a propeptide sequence.

The human and macaque PLA2G1B proteins may be present under two distinct forms: a pro form (proPLA2G1B), which is activated by proteolytic cleavage of a propeptide, leading to the mature secreted form (PLA2G1B). The term PLA2G1B includes any form of the protein, such as the pro-form and/or the mature form. Typically, the mature secreted forms comprise the sequence of amino acid residues 23-148 of SEQ ID NO: 37 and 38, or any variants thereof.

The term "antibody" designates any immunoglobulin-type molecule comprising heavy and light chains. Typical antibodies possess the basic monomeric "H2L2" structure consisting of 2 Heavy and 2 Light chains. Each heavy chain is paired with a light chain. Heavy and light chains comprise a "variable region" or "variable domain". The variable domain of the heavy chain may be referred to as "VH." The variable domain of the light chain may be referred to as "VL." These domains are generally the most variable parts of an antibody and contain the antigen-binding sites. A light or heavy chain variable region (VL or VH) consists of a framework region interrupted by three hypervariable regions referred to as "complementarity determining regions" or "CDRs".

Antibody "fragments" or "antigen-binding fragments" designate any portion of an antibody that confers antigen binding. Fragments typically designate Fab, Fab'2, ScFv, nanobodies, or CDR.

In preferred embodiments, antibodies, or antigen-binding fragments thereof, are isolated. An "isolated" antibody is one which has been separated from a component of its natural environment. In particular, the antibody may be purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

Antibody "variant", as used herein, refers to an antibody which retains the antigenic specificity of a reference antibody but wherein one or more amino acid residues are (e.g., chemically, or biologically) modified, typically to improve its properties. Examples of such chemical modifications include, e.g., by alkylation, PEGylation, acylation, ester or amide formation, and the like. In particular, a variant is an antibody as disclosed herein that is modified to contain one or more additional non-proteinaceous moieties such as water-soluble polymers. Examples of water-soluble polymers include, but are not limited to, PEG, copolymers of ethylene glycol/propylene glycol, carboxymethylcellulose, dextran and polyvinyl alcohol. Variants may also be generated to increase or decrease the extent to which the antibody is glycosylated. Addition or deletion of glycosylation sites to an antibody may be conveniently accomplished by altering the amino acid sequence such that one or more glycosylation sites is created or removed.

An antibody is "selective" for an antigen, or "selectively binds" an antigen when such antibody exhibits preferential binding to said antigen as compared to other molecules. Selectivity can be determined by competitive ELISA or BIAcore assays. The difference in affinity/avidity that marks selectivity can be any detectable preference, such as e.g., a ratio of more than 1:1.1, or more than about 1:5, 1:10, 1:100, 1:1000 or more.

The term "competitively inhibits" indicates that the antibody can reduce or inhibit or displace the binding of a reference antibody to PLA2G1B. Competition assays can be performed using standard techniques such as, for instance, competitive ELISA or other binding assays. Typically, a competitive binding assay involves a purified target antigen, generally bound either to a solid substrate or cells, an unlabeled test antibody and a labeled reference antibody. Competitive inhibition is measured by determining the amount of labeled antibody bound in the presence of the test antibody. Usually, the test antibody is present in excess, such as about 5 to 500 times the amount of reference antibody. Typically, for ELISA, the test antibody is in 100X excess, and for enzymatic methods, the test antibody in in 10X excess. When a test antibody present in excess inhibits or displaces at least 70% of the binding of the reference antibody to the antigen, it is considered as competitively inhibiting said reference antibody. In a specific embodiment, when a test antibody present in 100X excess inhibits or displaces at least 70%, more preferably at least 80% of the binding of the reference antibody to the antigen in ELISA, it is considered as competitively inhibiting said reference antibody. Preferred competing antibodies bind epitopes that share common amino acid residues.

### Anti-PLA2G1B antibodies

In a particular aspect, the invention relates to monoclonal antibodies, or fragments thereof, that bind to mammalian PLA2G1B. Preferably, monoclonal antibodies of the invention bind to human or simian PLA2G1B.

Such antibodies were generated by immunization of OF1 mice with recombinant human active PLA2G1B conjugated to BSA, as detailed in the experimental part. The antibodies were further selected for their capacity to inhibit PLA2G1B enzymatic activity using human and/or simian PLA2GIB. The amino acid sequences of the heavy and light chains of antibodies of the invention were determined, allowing recombinant production thereof and generation of variants thereof.

The amino acid sequences of the light and heavy chains variable regions of monoclonal antibodies of the invention are provided in SEQ ID NOs: 1, 5, 9, 13 and in Figures 5 and 6. The CDR domains of each variable region are also identified and provided as SEQ ID Nos: 21-32.

In a particular embodiment, the invention relates to a monoclonal antibody, or an antigen-binding fragment thereof, wherein said antibody or fragment thereof comprises a light chain variable region comprising (i) a CDR1 comprising, consisting, or consisting essentially of SEQ ID NO: 21, and/or (ii) a CDR2 comprising, consisting, or consisting essentially of SEQ ID NO: 22, and/or (iii) a CDR3 comprising, consisting, or consisting essentially of SEQ ID NO: 23.

In a further particular embodiment, the invention relates to a monoclonal antibody, or an antigen-binding fragment thereof, wherein said antibody or fragment thereof comprises a heavy chain variable region comprising (i) a CDR1 comprising, consisting, or consisting essentially of SEQ ID NO: 24, and/or (ii) a CDR2 comprising, consisting, or consisting essentially of SEQ ID NO: 25, and/or (iii) a CDR3 comprising, consisting, or consisting essentially of SEQ ID NO: 26.

In a further particular embodiment, the invention relates to a monoclonal antibody or fragment, wherein said antibody or fragment comprises a light chain variable region comprising SEQ ID NO: 1.

In a further particular embodiment, the invention relates to a monoclonal antibody or fragment, wherein said antibody or fragment comprises a heavy chain variable region comprising SEQ ID NO: 5.

In a further particular embodiment, the invention relates to a monoclonal antibody or fragment comprising a light chain variable region comprising SEQ ID NO: 1 and a heavy chain variable region comprising SEQ ID NO: 5.

In a further particular embodiment, the invention relates to a monoclonal antibody or fragment comprising a light chain sequence of SEQ ID NO: 18.

In a further particular embodiment, the invention relates to a monoclonal antibody or fragment comprising a heavy chain sequence of SEQ ID NO: 20.

In a further particular embodiment, the invention relates to a monoclonal antibody or fragment comprising a light chain sequence of SEQ ID NO: 18 and a heavy chain sequence of SEQ ID NO: 20.

In a further particular embodiment, the invention relates to a monoclonal antibody or an antigen-binding fragment thereof, wherein said antibody or fragment competes for binding to a mammalian PLA2G1B with an antibody comprising a light chain sequence of SEQ ID NO: 18, and/or a heavy chain sequence of SEQ ID NO: 20.

In a further particular embodiment, the invention relates to a monoclonal antibody, wherein said antibody or fragment competes with monoclonal antibody mAb1 for binding to PLA2G1B protein.

In a further particular embodiment, the invention relates to a monoclonal antibody, wherein said antibody or fragment comprises a light chain comprising a sequence selected from SEQ ID NO: 1, 9 and 18 and/or a heavy chain comprising a sequence selected from SEQ ID NO: 5, 13 and 20.

In a further particular embodiment, the invention relates to a monoclonal antibody, wherein said antibody comprises:
(i) a light chain comprising SEQ ID NO: 1 or 18 and/or a heavy chain comprising SEQ ID NO: 5 or 20; or
(ii) a light chain comprising SEQ ID NO: 9 and/or a heavy chain comprising SEQ ID NO: 13.

A particular example of such antibodies is mAb1 antibody, or an antigen-binding fragment thereof, or a variant thereof.

Another specific example of antibodies of the invention is 22C6 antibody, or an antigen-binding fragment or a variant thereof, and specific uses thereof as described herein.

Interactions of mAb1 and 22C6 antibodies with human and macaque active PLA2G1B and proPLA2G1B were studied by the inventors. The results obtained in the experimental part, demonstrate that mAb1 and 22C6 antibodies specifically label various human and simian tissues (such as pancreas) and are specific of active human and macaque PLA2G1B.

To determine concentrations of mAb1 and 22C6 antibodies that specifically label exocrine part of cynomolgus macaque pancreas, cynomolgus macaque pancreas (positive tissue) and muscle (negative tissue) were labelled with different amounts of mAb1 or 22C6 antibody for several times. As shown in the experimental part of the application, the inventors have found that with the concentration of about 0.1 µg/ml or more of mAb1 and with the concentration of about 0.2 µg/ml or more of 22C6, macaque PLA2G1B is primarily detected in the exocrine part of the cynomolgus macaque pancreas (Figure 2) with the highest specificity for active macaque PLA2G1B at about 0.2 µg/ml of mAb1 and of 22C6.

To determine concentrations of mAb1 and 22C6 that specifically label human active PLA2G1B in tissues, frozen human pancreas (positive tissue) and muscle (negative tissue) were labelled with different amounts of mAb1 or 22C6 antibody. The inventors have found that mAb1 and 22C6 preferentially labeled human exocrine pancreas at the concentration of at least 0.1 µg/ml, preferably, from 0.1 to 0.2 µg/ml or more (Figure 3). No signal was observed on muscle under these experimental conditions. This demonstrates that these conditions preferentially detect active human PLA2G1B in tissues with the highest specificity for active PLA2G1B at about 0.1 µg/ml.

To check the specificity for active PLA2G1B of mAb1 and 22C6 concentrations that specifically label exocrine part of cynomolgus and human pancreas, these labelling conditions were tested on CHO cells that express either human active PLA2G1B, or human proPLA2G1B or not transfected. mAb1 and 22C6 preferentially label active PLA2G1B than proPLA2G1B with the highest difference at about 0.2 and 0.1 µg/ml (Figure 4). These results show that these experimental conditions preferentially labelled active human PLA2G1B and show that 22C6 and mAb1 bind preferentially to active human and macaque PLA2G1B.

The inventors have further observed that mAb1 was very efficiently binding to human and cynomolgus macaque active PLA2G1B and with a much higher affinity for active PLA2G1B than proPLA2G1B in both species (Table 1). Half-life of the mAb1 Fab/hPLA2G1B complex is almost 4h (t ½ = 226 min) indicating that mAb1 Fab/hPLA2G1B complex is quasi-irreversible. mAb1 Fab affinity for hPLA2G1B is extremely high with a Kd of 6 pM. mAb1 Fab affinity is 273-fold higher for hPLA2G1B than hproPLA2G1B. mAb1 affinity is also high for macPLA2G1B with a Kd of 34pM. mAb1 Fab affinity is 1409-fold higher for macPLA2G1B than macproPLA2G1B (Table 6). These results with mAb1 Fab confirmed the high affinity of mAb1 monoclonal antibody (mAb) for active PLA2G1B in both human and simian species observed with mAb1 antibodies.

To study the effect of 22C6 and mAb1 antibodies on active human and macaque PLA2G1B enzymatic activity, recombinant PLA2G1B were preincubated with several concentrations of antibodies and then enzymatic activity was evaluated in the tritiated oleic acid release assay using radiolabeled *E. Coli* membranes (Figure 7). Both monoclonal antibodies are potent inhibitors of active PLA2G1B from human and macaque. The 22C6 IC50 was 58 pM on human PLA2G1B and 430 pM on macPLA2G1B. The mAb1 IC50 was 78 pM on human PLA2G1B and 590 pM on macaque PLA2G1B. These results indicate that 22C6 and mAb1 are very potent antibodies to block PLA2G1B activity in human and macaque. Thus, they are therapeutic candidates to block adverse effect of PLA2G1B in HIV patients (Pothlichet et al., 2020) and can also be used to control SIV or SHIV infection and adverse effect in cynomolgus monkeys.

In conclusion, the high affinity of mAb1 and 22C6 antibodies and their inhibitory activity on human and macaque PLA2G1B indicate that injection of these antibodies in human or macaque can be a new approach to restore CD4 T-cell function and may impair the onset of various immunodeficiency diseases, in particular in HIV-, SIV- or SHIV-infected subjects.

### Nucleic acids, vectors and host cells

The invention also relates to nucleic acids encoding an antibody, or a fragment thereof, as defined above, as well as cloning or expression vectors containing such nucleic acids, and recombinant host cells.

In another aspect, the present invention concerns nucleic acid molecules encoding an antibody of the invention, or a nucleic acid complementary to said encoding sequence. Preferably, the nucleic acid is an isolated or purified nucleic acid.

The nucleic acid can be DNA (cDNA or gDNA), RNA, or a mixture thereof. It can be in single stranded form or in duplex form or a mixture of the two. It can comprise modified nucleotides, comprising for example a modified bond, a modified purine or pyrimidine base, or a modified sugar. It can be prepared by any method known to one skilled in the art, including chemical synthesis, recombination, and mutagenesis. The nucleic acid according to the invention may be deduced from the sequence of the antibody according to the invention and codon usage may be adapted according to the host cell in which the nucleic acid shall be transcribed. These steps may be carried out according to methods well known to one of skill in the art and some of which are described in the reference manual Sambrook et al. (Sambrook J, Russell D (2001) Molecular cloning: a laboratory manual, Third Edition Cold Spring Harbor).

The nucleic acid of the invention may encode an amino acid sequence comprising the light chain and/or an amino acid sequence comprising the heavy chain of the antibody, or may be complementary to such encoding sequence.

Specific examples of such nucleic acid sequences include the sequences comprising any one of SEQ ID NOs: 3, 7, 11, 15, 17, 19, and the complementary sequence thereto. The present invention further provides a vector comprising a nucleic acid of the invention. Optionally, the vector may comprise several nucleic acids of the invention. In particular, the vector may comprise a nucleic acid of the invention operably linked to a regulatory region, i.e., a region comprising one or more control sequences. Optionally, the vector may comprise several nucleic acids of the invention operably linked to several regulatory regions.

The term "control sequences" means nucleic acid sequences necessary for expression of a coding region. Control sequences may be endogenous or heterologous. Well-known control sequences and currently used by the person skilled in the art will be preferred. Such control sequences include, but are not limited to, promoter, signal peptide sequence and transcription terminator.

The term "operably linked" means a configuration in which a control sequence is placed at an appropriate position relative to a coding sequence, in such a way that the control sequence directs expression of the coding region.

The present invention further relates to the use of a nucleic acid or vector according to the invention to transform, transfect or transduce a host cell.

The present invention also provides a host cell comprising one or several nucleic acids of the invention and/or one or several vectors of the invention.

The term "host cell" also encompasses any progeny of a parent host cell that is not identical to the parent host cell due to mutations that occur during replication. Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments *in E. coli*.) After expression, the antibody may be isolated from the bacterial cell lysate in a soluble fraction and can be further purified. In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22: 1409-1414 (2004), and Li et al., Nat. Biotech. 24:210-215 (2006). Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells. Plant cell cultures can also be utilized as hosts. See, e.g., US Patent Nos. 5,959,177, 6,040,498, 6,420,548, 7,125,978, and 6,417,429. Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse Sertoli cells (TM4 cells as described, e.g., in Mather, Biol. Reprod. 23:243-251 (1980)); monkey kidney cells (CV1); African green monkey kidney cells (VERO- 76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather et al., Annals N. Y. Acad. Sci. 383:44-68 (1982); MRC 5 cells; and FS4 cells.

Other useful mammalian host cell lines include Chinese hamster ovary (CHO) cells, including DHFR- CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255- 268 (2003).

The present invention also concerns a method for producing an antibody of the invention, comprising culturing a host cell comprising a nucleic acid of the invention or a vector of the invention, as provided above, under conditions suitable for expression of the antibody, and optionally recovering the antibody from the host cell or from the host cell culture medium. Optionally, the recovered antibody may be further purified or isolated. Suitable media, culture conditions and production method are well-known by the skilled person and can be easily chosen according to the host cell and the antibody to be produced.

### Pharmaceutical and diagnostic compositions

The present invention further relates to pharmaceutical or diagnostic compositions comprising an antibody, a nucleic acid, a vector or a host cell of the invention. The composition may comprise one or several antibodies of the invention, one or several nucleic acids of the invention and/or one or several vectors of the invention and/or one or several host cells of the invention. Preferably, the composition comprises one or several antibodies of the invention.

Pharmaceutical compositions comprise an antibody of the invention and any suitable excipient. Typically, the antibody has the desired degree of purity and is mixed with optional physiologically acceptable carriers, vehicle or stabilizers (Remington: The Science and Practice of Pharmacy 20th edition (2000)), in the form of e.g., aqueous solutions, lyophilized or other dried formulations.

Acceptable carriers, excipients, or stabilizers are nontoxic to recipients at the dosages and concentrations employed, and include, but are not limited to, buffering agents, stabilizing agents, preservatives, isotonifiers, non-ionic detergents, antioxidants and other miscellaneous additives. Buffering agents help to maintain the pH in the range which approximates physiological conditions. They are preferably present at concentration ranging from about 1 mM to about 50 mM. Suitable buffering agents for use with the present invention include, but are not limited to, both organic and inorganic acids and salts thereof such as citrate, succinate, tartrate, fumarate, gluconate, oxalate, lactate and acetate buffers, as well as phosphate buffers, histidine buffers and trimethylamine salts such as Tris. Preservatives may be added to retard microbial growth, and may be added in amounts ranging from 0.2%-1% (w/v). Suitable preservatives for use with the present invention include, but are not limited to, phenol, butyl or benzyl alcohol; meta-cresol; alkyl parabens such as methyl or propyl paraben; octadecyldimethylbenzyl ammonium chloride, benzalkonium halides (e.g., chloride, bromide, iodide); hexamethonium or benzethonium chloride; catechol; resorcinol; cyclohexanol; and 3-pentanol. Isotonifiers may be added to ensure isotonicity of liquid compositions of the present invention and include polyhydric sugar alcohols, preferably trihydric or higher sugar alcohols, such as glycerin, erythritol, arabitol, xylitol, sorbitol and mannitol. Stabilizing agents refer to a broad category of excipients which can range in function from a bulking agent to an additive which solubilizes the therapeutic agent or helps to prevent denaturation or adherence to the container wall. Typical stabilizers can be polyhydric sugar alcohols (enumerated above); amino acids such as arginine, lysine, glycine, glutamine, asparagine, histidine, alanine, ornithine, L-leucine, 2-phenylalanine, glutamic acid, threonine, etc., organic sugars or sugar alcohols, such as lactose, trehalose, stachyose, mannitol, sorbitol, xylitol, ribitol, myoinisitol, galactitol, glycerol and the like, including cyclitols such as inositol; polyethylene glycol; amino acid polymers; sulfur containing reducing agents, such as urea, glutathione, thioctic acid, sodium thioglycolate, thioglycerol, alpha-monothioglycerol and sodium thiosulfate; low molecular weight polypeptides (i.e. <10 residues); proteins such as human serum albumin, bovine serum albumin, gelatin or immunoglobulins; hydrophylic polymers, such as polyvinylpyrrolidone monosaccharides, such as xylose, mannose, fructose, glucose; disaccharides such as lactose, maltose, sucrose and trisaccharides such as raffinose; polysaccharides such as dextran. Stabilizers may be present in the range from 0.1 to 10,000 weights per part of weight therapeutic agent. Non-ionic surfactants or detergents (also known as "wetting agents") may be added to help solubilize the therapeutic agent as well as to protect the therapeutic protein against agitation-induced aggregation. Suitable non-ionic surfactants include, but are not limited to, polysorbates (20, 80, etc.), polyoxamers (184, 188 etc.), PLURONICS^{™}, polyols, polyoxyethylene sorbitan monoethers (TWEEN^{™}-20, TWEEN^{™}-80, etc.). Non-ionic surfactants may be present in a range of about 0.05 mg/ml to about 1.0 mg/ml, preferably about 0.07 mg/ml to about 0.2 mg/ml. Additional miscellaneous excipients include, but are not limited to, bulking agents (e.g., starch), chelating agents (e.g., EDTA), antioxidants (e.g., ascorbic acid, methionine, vitamin E), and cosolvents.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin micropheres, microemulsions, nano-particles and nanocapsules) or in macroemulsions. Such techniques are disclosed in Remington: The Science and Practice of Pharmacy 20th edition (2000).

The pharmaceutical compositions of the invention can be formulated for a topical, oral, parenteral, intranasal, intravenous, intramuscular, subcutaneous or intraocular administration and the like.

Preferably, the pharmaceutical formulation is a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. Sterile injectable solutions may be prepared by incorporating the active compounds in the required amount in the appropriate solvent with various of the other ingredients enumerated above, as required, followed by filtered sterilization. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

In addition to the compositions formulated for parenteral administration, such as intravenous or intramuscular injection, other pharmaceutically acceptable forms include, e.g. tablets or other solids for oral administration; time release capsules; and any other form currently used.

The pharmaceutical composition of the invention may comprise one or several antibodies of the invention.

The pharmaceutical composition may further comprise one or several additional active compounds. Examples of additional active compounds include, but are not limited to, antiviral agents, antibacterial agents, antifungal agents, antiparasitic agents or chemotherapeutic drugs.

The amount of antibody of the invention which will be effective in the treatment of a particular disorder or condition will depend on the nature of the disorder or condition, and can be determined by standard clinical techniques. In a preferred embodiment, each dose may range from about 0.1 mg to about 25 mg per kilogram of body weight of antibody, or more preferably, from about 1 mg to about 10 mg per kilogram body weight. The dosing schedule for administration may vary from once a month to daily depending on a number of clinical factors, including the type of disease, severity of disease, and the subject's sensitivity to the therapeutic agent.

### Methods of detection, dosage, diagnosis

In a further aspect, the present invention relates to a method for detecting or quantifying PLA2G1B protein in a sample, using an antibody or fragment as defined above. The method can be used or implemented for monitoring the severity or progression of HIV, SIV or SHIV infection in a subject, as well as for predicting the outcome of such infection or determining the optimal treatment.

In a particular embodiment, the method comprises providing a sample, and detecting the presence or amount of PLA2G1B protein in the sample using an antibody or fragment as defined above.

Typically, the detecting step b) comprises the steps of (i) contacting the sample, or biological replicates of the sample, with any one of the monoclonal antibodies or fragments thereof herein described, and (ii) detecting the presence of an antibody-antigen complex formed in step (i).

Detecting the presence of an antibody-antigen complex can be performed by any technique well known *per se* by the skilled person and extensively described in the literature. Typically, detection can be performed with a second antibody against PLA2G1B, preferably that binds an epitope distinct (and remote) from that of the first capture antibody. Alternatively, the capture antibody may be labelled and the detection step may comprise measuring the amount of label in the reaction, typically after removing unbound antibodies.

The antibody may be immobilized on a surface or particle, or not. The reaction can be performed in any appropriate support or container, such as microplate, on a chip, in a bottle, etc. Also, in a particular embodiment, the detecting step (ii) utilizes a labelled antibody, such as an antibody conjugated to a reporting group or engineered to be detectable, more preferentially said antibody is biotinylated.

The method may be used with any sample such as any biological sample, such as a serum sample, a plasma sample, whole blood, biological fluids, etc. In more particular embodiments, the sample used is obtained from a subject. The sample may be treated prior to detection, such as by dilution, concentration, enrichment, etc. It may be lyophilized, sterilized, frozen, etc., before use in a method of the invention.

In a particular embodiment, the invention thus relates to a method for detecting a PLA2G1B protein in a sample, comprising:
(i) contacting the sample with a monoclonal antibody, variant or fragment of the invention, and
(ii) detecting the presence of an antibody-antigen complex formed in step (i).

The invention is particularly relevant since the antibodies of the invention can detect or quantify the active form of PLA2G1B.

The invention thus relates to a method for monitoring progression of HIV, SIV or SHIV disease in a subject in need thereof, comprising *in vitro* quantifying PLA2G1B at different time intervals in a plasma sample from the subject using a monoclonal antibody or fragment of the invention.

The method of the invention may be used e.g., for monitoring anti-HIV, anti-SIV or anti-SHIV treatment efficacy.

The method of the invention may also be used for determining if an HIV-, SIV- or SHIV-infected subject can benefit from anti-PLA2G1B antibody therapy or trans CD4 immunotherapy.

The method of the invention can also be used for determining the severity of a disease caused by HIV, SIV or SHIV, wherein an increased level of binding of the monoclonal antibody or fragment of the invention in said sample, as compared to a reference value, is indicative of severity. For use in the invention, the reference value may be a value determined at an earlier stage in a sample from the same subject, or a mean value.

The invention mays also be used to monitor/follow the amount of PLA2G1B in the plasma of HIV-, SIV- or SHIV-infected subjects treated with antiretroviral molecules and correlate the PLA2G1B levels and activity with outcome of the disease.

In a further aspect, the present invention relates to kits comprising a least one antibody, or a fragment thereof, as herein described, in a container (such as a plate, microplate, tube, bottle, flask, etc. Preferably, the kit of the invention further comprises one or more reagent(s) to perform, detect or quantify an immune reaction, or an antibody-antigen complex. The one or more reagents may be selected from e.g., diluents, substrates, labels, marker antibodies, etc., adapted to any immune reaction such as ELISA, RIA, etc.

### Methods of treatment

The antibodies and compositions of the present invention, in particular mAb1 antibodies, may be used for the treatment of any immunodeficiency or an associated disease. As used herein, "immunodeficiency or an associated disease" designates any condition or pathology characterized by and/or caused by a reduced immune function or response in a subject. In particular, immunodeficiency or an associated disease may be cancer or an infectious disease (caused by any pathogen, e.g., bacterium or virus such as HIV, hepatitis B, etc.). More specifically, the antibodies and compositions of the present invention may be used for the treatment of any immunodeficiency associated with an activity of PLA2G1B, and, in particular associated with an inappropriate activity of CD4 T cells. In this regard, the inventors have previously characterized that PLA2G1B is a key endogenous factor of the immune response and that PLA2G1B plays a crucial role in the mechanism underlying the unresponsiveness of CD4 T lymphocytes observed for example in viremic HIV-infected patient (WO2015/09714, WO2019/166664). The invention is thus particularly suitable for treating immunodeficiencies or associated diseases related to CD4 T cells. In a particular embodiment, the invention is therefore directed to methods for treating CD4 cells related immunodeficiencies or associated diseases, by using an antibody of the invention, such as mAb1 antibody, and namely by inhibiting PLAG1B.

### Cancer

The invention also provides methods, compositions and antibodies, such as mAb1, for treating cancer. In a particular embodiment, the invention also relates to an antibody, such as mAb1, for use for preventing cancer or reducing the rate of cancer occurrence in a subject in need thereof. In this regard, the invention can be used for treating risk factors for cancers, thereby avoiding or reducing the risk/rate of occurrence of a cancer. Such risk factors include, without limitation, oro-, gastro-, and/or intestinal inflammation and infections, such as pancreatitis.

In another particular embodiment, the invention relates to an antibody, such as mAb1, for use for reducing the rate of cancer progression in a subject having a cancer. In another particular embodiment, the method of the invention is for reducing or preventing or treating cancer metastasis in a subject having a cancer, or for killing cancer cells. In another particular embodiment, the invention relates to an antibody, such as mAb1, for use for reducing or preventing or treating cancer metastasis in a subject having a cancer, or for killing cancer cells in a subject having a cancer.

The invention may be used for treating any cancer. In a particular embodiment, the cancer is a solid cancer. The invention is particularly suitable for treating cancers or neoplasia in subjects having a PLA2G1B-related CD4 T cell immunodeficiency. The invention may be used to treat cancers at any stage of development. In this regard, most solid cancers develop through four stages:
Stage I. This stage is usually a small cancer or tumor that has not grown deeply into nearby tissues. It also has not spread to the lymph nodes or other parts of the body. It is often called early-stage cancer.
Stage II and Stage III. In general, these 2 stages indicate larger cancers or tumors that have grown more deeply into nearby tissue. They may have also spread to lymph nodes but not to other parts of the body.
Stage IV. This stage means that the cancer has spread to other organs or parts of the body. It may also be called advanced or metastatic cancer.

Some cancers also have a stage 0. Stage 0 cancers are still located in the place they started and have not spread to nearby tissues. This stage of cancer is often highly curable, usually by removing the entire tumor with surgery.

The invention may be used for treating tumors or cancers at stage 0, I, II, III or IV. The invention may be used to prevent or reduce or treat metastasis of a cancer at stage 0, I, II or III. The invention may be used to reduce the rate of progression of a cancer at stage 0, I, II, III or IV. The invention may in particular be used for treating solid cancers selected from pancreatic cancer, melanoma, lung, oesophageal or pharyngeal cancer, retinoblastoma, liver, breast, ovary, renal, gastric, duodenum, uterine, cervical, thyroid, bladder, prostate, bone, brain or colorectal cancer.

### Infectious diseases

In further embodiments, the present invention relates to an antibody, such as mAb1 antibody, for treating an infectious disease caused, for example, by bacterial, viral, fungal or parasite infection.

Examples of infectious bacteria that can be treated with the methods, compositions or antibodies provided herein include, but are not limited to, any type of Gram-positive (such as Streptococcus, Staphylococcus, Corynebacterium, Listeria, Bacillus and Clostridium) or Gram-negative bacteria (such as Porphyromonas, Salmonella, Shigella, Enterobacteriaceae, Pseudomonas, Moraxella, Helicobacter, Stenotrophomonas, acetic acid bacteria, alpha-proteobacteria, Escherichia coli, Neisseria gonorrhoeae, Neisseria meningitidis, Moraxella catarrhalis, Hemophilus influenzae, Klebsiella pneumoniae, Legionella pneumophila, Pseudomonas aeruginosa, Proteus mirabilis, Enterobacter cloacae and Serratia marcescens. Exemplary infectious bacteria include, but are not limited to: Porphyromonas gingivalis, Porphyromonas somerae, Terrisporobacter glycolicus, Aggregatibacter actinomycetemcomitans, Aggregatibacter aphrophilus, Bacteroides fragilis, Helicobacter pyloris, Borelia burgdorferi, Legionella pneumophilia, Mycobacteria sps (such as M. tuberculosis, M. avium, M. intracellulare, M. kansaii, M. gordonae), Staphylococcus aureus, Neisseria gonorrhoeae, Neisseria meningitidis, Listeria monocytogenes, Streptococcus pyogenes (Group A Streptococcus), Streptococcus agalactiae (Group B Streptococcus), Streptococcus (viridans group), Streptococcus faecalis, Streptococcus bovis, Streptococcus (anaerobic sps.), Streptococcus pneumoniae, pathogenic Campylobacter sp., Enterococcus sp., Haemophilus influenzae, Bacillus anthracis, corynebacterium diphtheriae, corynebacterium sp., Erysipelothrix rhusiopathiae, Clostridium perfringers, Clostridium tetani, Enterobacter aerogenes, Klebsiella pneumoniae, Pasturella multocida, Bacteroides sp., Fusobacterium nucleatum, Streptobacillus moniliformis, Treponema pallidium, Treponema pertenue, Leptospira, and Actinomyces israelli.

Examples of infectious fungi that can be treated with the methods, compositions or antibodies provided herein include, but are not limited to, Cryptococcus neoformans, Histoplasma capsulatum, Coccidioides immitis, Blastomyces dermatitidis, Chlamydia trachomatis, Candida albicans and Candida glabrata.

Examples of infectious parasites that can be treated with the methods, compositions or antibodies provided herein include, but are not limited to Plasmodium falciparum and Toxoplasma gondii.

The invention may also be used to treat viral diseases or viral infection in mammals. Examples of viruses that can be treated with the methods, compositions or antibodies provided herein include, but are not limited to, enveloped viruses such as members of the following viral families: Retroviridae (e.g., HIV (such as HIV1 and HIV2), MLV, SIV, FIV, Human T-cell leukemia viruses 1 and 2, XMRV, and Coltiviruses (such as CTFV or Banna virus)); Togaviridae (for example, alphaviruses (such as Ross River virus, Sindbis virus, Semliki Forest Virus, O'nyong'nyong virus, Chikungunya virus, Eastern equine encephalitis virus, Western equine encephalitis virus, Venezuelan equine encephalitis virus) or rubella viruses); Flaviridae (for example, dengue viruses, encephalitis viruses (such as West Nile virus or Japanese encephalitis virus), yellow fever viruses); Coronaviridae (for example, coronaviruses such as SARS virus, MERS-CoV, SARS-CoV-2 or Toroviruses); Rhabdoviridae (for example, vesicular stomatitis viruses, rabies viruses); Paramyxoviridae (for example, parainfluenza viruses, mumps virus, measles virus, respiratory syncytial virus, sendai virus, and metopneumovirus); Orthomyxoviridae (for example, influenza viruses); Bunyaviridae (for example, Hantaan virus, bunya viruses (such as La Crosse virus), phleboviruses, and Nairo viruses); Hepadnaviridae (Hepatitis B viruses); Herpesviridae (herpes simplex virus (HSV) 1 and HSV-2, varicella zoster virus, cytomegalovirus (CMV), HHV-8, HHV-6, HHV-7, and pseudorabies virus); Filoviridae (filoviruses including Ebola virus and Marburg virus) and Poxyiridae (variola viruses, vaccinia viruses, pox viruses (such as small pox, monkey pox, and Molluscum contagiosum virus), yatabox virus (such as Tanapox and Yabapox)). Non-enveloped viruses can also be treated with the methods provided herein, such as members of the following families: Calciviridae (such as strains that cause gastroenteritis); Arenaviridae (hemorrhagic fever viruses such as LCMV, Lassa, Junin, Machupo and Guanarito viruses); Reoviridae (for instance, reoviruses, orbiviruses and rotaviruses); Birnaviridae; Parvoviridae (parvoviruses, such as Human bocavirus adeno-associated virus); Papillomaviridae (such as papillomaviruses); Papovaviridae (papilloma viruses, polyoma viruses); Adenoviridae (adenoviruses); Picornaviridae (enteroviruses, enteric viruses, Poliovirus, coxsackieviruses, hepatoviruses, cardioviruses, aptoviruses, echoviruses, hepatitis A virus, Foot and mouth disease virus, and rhinovirus) and Iridoviridae (such as African swine fever virus). Other viruses that can be treated using the methods provided herein include unclassified viruses (for example, the etiological agents of Spongiform encephalopathies, the agent of delta hepatitis (thought to be a defective satellite of hepatitis B virus), the agents of non-A, non-B hepatitis (for instance, Hepatitis C); calciviruses (such as Norovirus, Norwalk and related viruses); Hepeviruses (such as Hepatitis E), JC and BK viruses and astroviruses).

In a particular embodiment, the invention relates to an antibody of the invention, such as mAb1, for use in the treatment of HIV infection. In some embodiments, the subject is a patient with low immunoreconstitution after antiretroviral treatment and/or with severe idiopatic CD4 T lymphopenia (ICL). The invention also relates to a method for increasing CD4-T cell activity in a HIV-infected subject by administering an antibody of the invention, such as mAb1, optionally in combination with another active ingredient, preferably a PLA2G1B inhibitor.

The invention may be used to treat subjects at an early stage of the infection, to prevent or reduce occurrence, extent, or duration of an immunodeficiency. Typically, the antibodies can be administered immediately upon detection of an infectious disease, and prior to appearance of clinical signs. Administered very early during infection by HIV, antibodies of the invention can lead the patients toward a HIV controller status. The antibodies of the invention may also be administered later in infected subjects, either alone or in combination with one or more antiviral agent(s). In such regimen, they accelerate the recovery of CD4 T lymphocytes and the restoration of their functions. Accordingly, in a particular embodiment, the invention comprises simultaneously, separately or sequentially administering to subject having a viral infection (i) an antiviral agent and (ii) an antibody of the invention such as mAb1. Such protocol is particularly suited for treating HIV-infected subjects, wherein the antibody is used in combination with antiretroviral therapy (e.g., HAART), allowing to reduce HAART or even to at least temporarily interrupt HAART treatment which is known for its severe detrimental effects.

In other particular embodiments, the antibodies and compositions of the invention may preferably be used for treating various types of immunodeficiencies such as an immunodeficiency caused by HIV, SIV or SHIV, in a subject in need thereof.

In a further embodiment, the antibodies of the invention may be used to restore CD4 T-cells function in a HIV-, SIV- or SHIV-infected subject.

In a further aspect, the invention relates to methods for treating a disease caused by HIV, SIV or SHIV, comprising administering to a subject in need thereof a composition or antibody, fragment or variant thereof as defined above.

In a further aspect, the invention relates to a composition or antibody, fragment or variant thereof as defined above for use for treating HIV-, SIV- or SHIV-induced disease.

In a further particular embodiment, the invention relates to a monoclonal antibody for use in the treatment of an immunodeficiency caused by HIV, SIV or SHIV, or for use to restore CD4 T-cells function in a HIV-, SIV- or SHIV-infected subject, wherein said antibody comprises a light chain comprising a sequence selected from any of SEQ ID NO: 1, 9 and 18 and/or a heavy chain comprising a sequence selected from any of SEQ ID NO: 5, 13 and 20.

In a further particular embodiment, the invention relates to a monoclonal antibody for use in the treatment of an immunodeficiency caused by HIV, SIV or SHIV, or for use to restore CD4 T-cells function in a HIV-, SIV- or SHIV-infected subject, wherein said antibody comprises a light chain comprising a sequence selected from SEQ ID NO: 1 or 18 and a heavy chain comprising a sequence of SEQ ID NO: 13.

In a further particular embodiment, the invention relates to a monoclonal antibody for use in the treatment of an immunodeficiency caused by HIV, SIV or SHIV, or for use to restore CD4 T-cells function in a HIV-, SIV- or SHIV-infected subject, wherein said antibody comprises a light chain comprising a sequence of SEQ ID NO: 9 and a heavy chain comprising a sequence selected from SEQ ID NO: 5 and 20.

In a further particular embodiment, the invention relates to a monoclonal antibody for use in the treatment of an immunodeficiency caused by HIV, SIV or SHIV, or for use to restore CD4 T-cells function in a HIV-, SIV- or SHIV-infected subject, wherein said antibody comprises a light chain comprising SEQ ID NO: 1 or 18 and/or a heavy chain comprising SEQ ID NO: 5 or 20.

In a further particular embodiment, the invention relates to a monoclonal antibody for use in the treatment of an immunodeficiency caused by HIV, SIV or SHIV, or for use to restore CD4 T-cells function in a HIV-, SIV- or SHIV-infected subject, wherein said antibody comprises a light chain sequence of SEQ ID NO: 18 and/or a heavy chain sequence of SEQ ID NO: 20.

In a further particular embodiment, the invention relates to a monoclonal antibody for use in the treatment of an immunodeficiency caused by SIV or SHIV, or for use to restore CD4 T-cells function in a SIV- or SHIV-infected subject, wherein said antibody comprises a light chain comprising a sequence selected from SEQ ID NO: 9 and/or a heavy chain comprising a sequence selected from SEQ ID NO: 13.

In a further particular embodiment, the invention relates to a monoclonal antibody for use in the treatment of an immunodeficiency caused by SIV or SHIV, or for use to restore CD4 T-cells function in a SIV- or SHIV-infected subject, wherein said antibody comprises a light chain variable region comprising (i) a CDR1 comprising, consisting, or consisting essentially, of a sequence selected from SEQ ID NO: 27, and/or (ii) a CDR2 comprising, consisting, or consisting essentially, of a sequence selected from SEQ ID NO: 28, and/or (iii) a CDR3 comprising, consisting, or consisting essentially, of a sequence selected from SEQ ID NO: 29.

In a further particular embodiment, the invention relates to a monoclonal antibody for use in the treatment of an immunodeficiency caused by SIV or SHIV, or for use to restore CD4 T-cells function in a SIV- or SHIV-infected subject, wherein said antibody comprises a heavy chain variable region comprising (i) a CDR1 comprising, consisting, or consisting essentially, of a sequence selected from SEQ ID NO: 30, and/or (ii) a CDR2 comprising, consisting, or consisting essentially, of a sequence selected from SEQ ID NO: 31, and/or (iii) a CDR3 comprising, consisting, or consisting essentially, of a sequence selected from SEQ ID NO: 32.

As used herein, "treatment" or "treat" refers to clinical intervention in an attempt to alter the natural course of the individual being treated, and can be performed either for preventive or curative purpose. Desirable effects of treatment include, but are not limited to, preventing occurrence or recurrence of disease, alleviation of symptoms, diminishment of any direct or indirect pathological consequences of the disease, reducing viral load or replication or virulence, decreasing the rate of disease progression, amelioration or palliation of the disease state, and remission or improved prognosis. In some embodiments, compositions and methods of the invention are used to delay development of a disease or disorder or to slow the progression of a disease or disorder.

An antibody of the invention can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

The duration, dosages and frequency of administering antibodies or compositions of the invention may be adapted according to the subject and disease, and will be dependent upon the age, health, sex, and weight of the subject, kind of concurrent treatment, if any, frequency of treatment, and the nature of the pharmaceutical effect desired. The treatment may be used alone or in combination with other active ingredients, either simultaneously or separately or sequentially. Further aspects and advantages of the invention are disclosed in the following experimental section.

### EXAMPLES

### MATERIALS AND METHODS

### Recombinant proteins and peptides

For immunization to generate anti-PLA2G1B and to select inhibitory anti-active PLA2G1B antibodies, human active PLA2G1B and proPLA2G1B were a gift from Gerard Lambeau (purity >98%, Genbank No. NP_000919.1) and produced in *E. coli* according to published procedures (Singer AG et al., J Biol Chem. 2002 Dec 13;277(50):48535-49; Rouault M et al., Biochemistry. 2007 Feb 13;46(6):1647-62). Briefly, the protein proPLA2G1B was produced in *E. coli* as inclusion bodies, then renatured. The propeptide of the proenzyme was cleaved by trypsine and purified in active form by reverse phase HPLC. The quality of the pure protein was validated by MS (MALDI-TOF) and the enzyme activity was checked according to published procedures (Rouault M et al., Biochemistry. 2007 Feb 13;46(6): 1647-62) using [3H]-oleate-radiolabeled autoclaved *E. coli* membranes as phospholipid substrate in sPLA2 activity buffer. To perform SPR experiments, to determine IC50 of PLA2G1B enzymatic activity by 22C6 and mAb1 antibodies and to study their effect on inhibition of pSTAT5 NT response to IL-7, human active PLA2G1B (purity>93% for pSTAT5 NT experiments and >97% for SPR and PLA2G1B enzymatic activity inhibition assay), proPLA2G1B (purity >99%), macaque active PLA2G1B (purity >98%, Gene accession No. XP_005572452.1) and macaque proPLA2G1B (purity >99%, Gene accession No. XP_005572452.1) were produced in CHO cells.

### Development of anti-PLA2G1B antibodies

Antibodies specific for active human PLA2G1B (SEQ ID NO: 37) were generated at BIOTEM (France) by immunization of OF1 mice with active recombinant human PLA2G1B (hPLA2G1B) conjugated to BSA. 5 female OF1 mice, purchased from Charles River laboratories and bred at BIOTEM animal facility (Grenoble, France), were used for immunization. Mice were 7-weeks old upon the first immunogen injection. At D0, they were intraperitoneally (IP) and subcutaneously (SC) immunized with an injection of 50 µg/site (Mice S1, S2, S3) or 20 µg/site (Mice S4, S5), of active hPLA2G1B conjugated to BSA mixed with Complete Freund's adjuvant (v/v), followed by 2 IP injections with 50 µg (Mice S1, S2, S3) or 20 µg (Mice S4, S5) of the corresponding hPLA2G1B protein mixed with Incomplete Freund's adjuvant at day 21 and day 41 after the first injection.

To select mice that produce antibodies specific of hPLA2G1B, serums from mice were collected 8 days after the third injection and were tested at BIOTEM by ELISA on unconjugated hPLA2G1B, hPLA2GX, hPLA2GIIA or hPLA2GIID. Serums from mice immunized with hPLA2G1B showed significant high levels of anti-PLA2G1B antibodies. Mouse S1 was selected according to the high reactivity against PLA2G1B and low against PLA2GIIA, PLA2GIID and PLA2GX. The reactivity of serum was confirmed by inventors using ELISA and enzymatic activity.

Mouse S1 was intravenously injected with 10 µg of active hPLA2G1B conjugated to BSA 53 days after the first immunization. Three days later the spleen was collected and cells were fused with myeloma (Sp2/0-Ag14) cells using polyethylene glycol to generate hybridomas. The resulting hybridomas were then cultured in a selective DMEM medium supplemented with L-Glutamine (4 mM), heat inactivated FCS (20%), HAT (Hypoxanthine-Aminopterin-Thymidine,1X), HES (HydroxyEthyl Starch 130, 2%) and Penicillin/Streptomycin (1%). Hybridomas were allowed to grow for at least 7 days in the selection medium for colony formation and antibody production. The production of antibodies that specifically recognize PLA2G1B was assessed by ELISA. Positive hybridomas were cloned and grown in order to identify single-cell-derived clones secreting monoclonal antibodies of interest. For the mouse S1: 332^{∗}10e⁶ splenocytes were fused to 66.4^{∗}10e⁶ myeloma cells (1/5) and 20 plates were seeded. 5 plates were seeded at 100000 cells/well (N1 to N5) while 15 plates were seeded at 50000 cells/well (N6 to N20). The resulting 1442 hybridomas were analyzed in function of their growth and their capacity to produce IgG and 62 were screened by ELISA on unconjugated hPLA2G1B, hPLA2GX, hPLA2GIIA or hPLA2GIID (see details below). Hybridomas were selected for a good affinity on hPLA2G1B and a low affinity on hPLA2GX, hPLA2GIIA or hPLA2GIID. Supernatant from hybridomas with specific affinity for PLA2G1B were sent to inventors to be tested for their enzymatic activity inhibition according to published procedures (Rouault M et al., Biochemistry. 2007 Feb 13;46(6): 1647-62). Total IgG of hybridomas with inhibitory activity supernatants were purified on protein-A sepharose beads from culture supernatant. These IgGs were used to test inhibition of hPLA2G1B enzymatic activity at a known concentration of antibody.

The cell media of the expanded hybridomas of interest were then re-screened by ELISA (BIOTEM) to identify stable hybridomas capable of secreting anti-PLA2G1B Ab specific for PLA2G1B i.e., with good affinity on hPLA2G1B and a low affinity on hPLA2GX, hPLA2GIIA or hPLA2GIID. Positive hybridomas were cloned and grown in order to identify single-cell-derived clones secreting monoclonal antibodies of interest. Furthermore, antibodies produced by single-cell-derived clones selected were purified with protein-A sepharose beads to test their specificity on active hPLA2G1B relatively to hproPLA2G1B. Binding affinities to proPLA2G1B and active PLA2G1B were measured by ELISA (see below). Using this procedure several cloned hybridomas were selected: 22C6, 34G3, 23C7, 23F10, 24B2, 28A1, 28A3 (as previously described in international application WO2019/166664), and mAb1.

### Inhibition of PLA2G1B enzymatic activity assay

Inhibition of active PLA2G1B enzymatic activity by the above monoclonal antibodies (mAb) was tested according to published procedures (Rouault M et al., Biochemistry. 2007 Feb 13;46(6):1647-62). Briefly, recombinant human or macaque active PLA2G1B (0.2 nM) were incubated with 10 or 50 µL of hybridomas cell culture supernatants or increasing amount (typically 0.1, 0.3, 1, and 3 µg) of a purified rabbit polyclonal immune-serum targeted towards human active PLA2G1B or of purified monoclonal antibodies for 30 min at 37°C. PLA2G1B enzymatic activity was then measured using [3H]-oleate-radiolabeled autoclaved *E. coli* membranes as phospholipid substrate in PLA2G1B activity buffer. Reactions were stopped by addition of 300 µL of stop buffer, mixtures were then centrifuged at 10000 x g for 5 min, and the supernatants containing released [3H]-oleate were counted. Results were analyzed with a 4-parameter fit analysis using Prism^{™} software (Graph Pad) and reported as IC50.

### ELISA on hPLA2G1B, hPLA2GX, hPLA2GIIA or hPLA2GIID

To screen hybridoma supernatants for PLA2G1B specificity ELISA were performed. Briefly, 96-well microplates were coated with unconjugated hPLA2G1B, hPLA2GX, hPLA2GIIA or hPLA2GIID proteins in PBS (100 µL/well), incubated overnight at RT and then blocked with 150 µL/well of PBS-dried skimmed milk for 1h at RT. After one wash with PBS tween 0.05%, hybridoma supernatants (pure or diluted at 1/10) were added (50 µL/well) for 2h at room temperature. Plates were washed three times and peroxidase (HRP) conjugated goat anti-Mouse IgG + IgM was added at 1/10000 in PBS-tween 0.05%, 1% BSA (50 µL/well) for 1h at RT. After three washes, plates were stained with TMB substrate (KPL, Gaithersburg, USA) and read at OD 450 nm.

### ELISA on human active PLA2G1B or proPLA2G1B

Microplate wells were typically coated with 100 ng of recombinant human active PLA2G1B or proPLA2G1B in PBS pH 7.5, overnight at 4°C. Sample wells were washed three times with PBS containing 0.05% Tween 20. After final washing, sample wells were treated with blocking solution containing 1% BSA in PBS buffer for 60 min at room temperature. Following washing with PBS containing 0.05% Tween 20, increasing amounts (10 ng/ml up to 3 µg/ml) of mAb directed against human proPLA2G1B or active PLA2G1B were added to antigen-coated wells and incubated for 2h at room temperature. Following washing with PBS containing 0.05% Tween 20, the binding of mAb was detected with a HRP-conjugated goat anti-mouse IgG or IgA polyclonal antibody reagent and developed using colorimetric substrate. The dependency of the signals on the concentrations of antigen in solution was analyzed with a 4-parameter fit analysis using Prism^{™} software (Graph Pad) and reported as EC50.

### Immunohistochemistry analysis of PLA2G1B protein expression

Immunohistological analyses were performed at Histalim (France) on frozen normal human and cynomolgus macaque tissues provided by Histalim. The procedure to specifically detect active PLA2G1B by immunohistochemistry with the tested antibodies was set-up using CHO cells that express only active human PLA2G1B relative to CHO cells that express human proPLA2G1B or those that were not transfected. The human donor and cynomolgus macaque tissues were qualified by performing hematoxylin-eosin (H&E) staining and vimentin immunohistochemistry on DAKO Autostainer 48 platform using Envision flex revelation kit. Immunohistochemistry analysis was performed using 20 µg/ml of purified Ab from hybridoma supernatants 34G3, 22C6, 23C7, 23F10, 24B2, 28A1, 28A3, mAb1 or mouse IgG2a (control isotype) on cynomolgus macaque pancreas and muscle (Figure 1).

Immunochemistry analysis with 22C6 and mAb1 purified antibodies on cynomolgus tissues (Figure 2), human tissues (Figure 3) and CHO cells overexpressing human active PLA2G1B, proPLA2G1B or untransfected (Figure 4) was performed on frozen sections around 5 µm thick prepared on a Leica cryostat deposed on Superfrost plus slides (stored at - 80°C), then thawed for 15 to 60 min, post-fixed in formol for 20 min, and incubated for 32 min at 37°C with several concentrations of mAb1, 22C6 or mouse IgG2a (control isotype) antibodies (0.1, 0.2, 0.5, 1 µg/ml on cynomolgus tissues and CHO cells; 0.1, 0.2 µg/ml on human tissues), using a Ventana Benchmark XT automated platform (Roche). Labelling with primary antibodies was revealed with ultraview DAB detection kit and Hematoxylin/bluing reagent counterstain. The complete stained slides were digitalized using a Nanozoomer scanner (HAMAMATSU) in bright field conditions, at x20 magnification, without Z stack.

### Sequencing and analysis of cDNA coding variable regions of the 22C6 and mAb1 antibodies

RNA was extracted from cloned hybridomas and reverse transcribed into cDNA at BIOTEM (France) with high fidelity reverse transcriptase. The cDNA was amplified by PCR with high fidelity Taq polymerase and degenerated primers flanking variable regions of light and heavy chains. Both strands of the bulk of PCR products were directly sequenced. Sequences were aligned to obtain sequences coding the variable domains of light (VL) and heavy (VH) chains with partial sequences of the constant domains for 22C6 and complete sequences of the constant domains for mAb1. Based on these nucleotide sequences, protein sequences were deduced and isotypes of light and heavy chains were confirmed. Using the IMGT web site, (the international ImMunoGeneTics information system^{®} http://www.imgt.org) the percentage of identity between anti-PLA2G1B mAb VL and VH sequences and mouse germinal sequences was determined, the functional structure subdomains FR1-FR4 and CDR1-3 and a model of representation of 3D structures in Colliers de Perles (IMGT Colliers de Perles according to Pommié et al., 2004) were predicted.

### Determination of 22C6, mAb1 antibodies and mAb1 Fab Kd towards human and macaque PLA2G1B by Surface Plasmon Resonance

All protein interactions were studied at 25°C in PBS BSA 0.1% buffer. Interactions of 22C6 and mAb1 antibodies with human and macaque active PLA2G1B and proPLA2G1B were studied using surface plasmon technology on a BIAcore^{™} T200 (GE Healthcare) and CM5 sensorchips with covalently bound polyclonal anti-mouse IgG (Mouse Antibody Capture Kit, GE Healthcare). Isotype control, 22C6 or mAb1 were first injected and then human or macaque active PLA2G1B or proPLA2G1B were added. Kinetic analysis using BIAevaluation^{™} software were performed to obtain the association (Kon) and dissociation (Koff) rate constants. Specific signals of antigen binding to antibodies were determined using two references: subtraction of background signal due to antigen binding to control isotype and subtraction of background signal due to injection of buffer alone on bound 22C6 or mAb1 antibody. Specific signals due to association phase and dissociation phase were then analyzed for each antigen concentration using a mathematic model of simple 1:1 interaction between antigen and mAb (Langmuir model), t ½ is half-life time of the antigen-antibody complex and equal to ln2/Koff and Kd is dissociation equilibrium constant or affinity constant and equal to Koff/Kon.

Interactions of recombinant mAb1 Fab with human and macaque active PLA2G1B and proPLA2G1B were similarly studied as mAbs with CM5 sensorchips covalently bound polyclonal anti-mouse Fab IgG (Anti-mouse antibody IgG (Fab specific), Sigma, cat#M6898, lot 018MV4866V). Specific signals of antigen binding to mAb1 Fab were determined using two references: subtraction of background signal due to antigen binding without bounded Fab and subtraction of background signal due to injection of antigen on bounded irrelevant Fab. Specific signals due to association phase and dissociation phase were then analyzed for each antigen concentration using a mathematic model of simple 1:1 interaction between antigen and Fab (Langmuir model).

### Purification of Human CD4 T-lymphocytes

Venous blood was obtained from healthy volunteers through the EFS (Etablissement Français du Sang, Centre Necker-Cabanel, Paris, France). CD4 T-cells were purified from whole blood using RosetteSep Human CD4+ T cell Enrichment Cocktail (Stem Cell, 15062). This cocktail contains mouse and rat monoclonal antibodies purified from mouse ascites fluid or hybridoma culture supernatant, by affinity chromatography using protein A or Protein G sepharose. These antibodies are bound in bispecific tetrameric antibody complexes which are directed against cell surface antigens on human hematopoietic cells (CD8, CD16, CD19, CD36, CD56 CD66b, TCRa/β) and glycophorin A on red blood cells. The RosetteSep antibody cocktail crosslinks unwanted cells in human whole blood to multiple red blood cells, forming immunorosettes. This increases the density of unwanted cells, such that they pellet along with the free red blood cells when centrifuged over a buoyant density medium such as lymphocytes separation medium (Eurobio, CMSMSL01-01).

Whole blood was incubated with RosetteSep Human CD4+ T cell Enrichment Cocktail at 50µL/ml for 20 minutes at room temperature under gentle shaking (100 rpm), diluted with an equal volume of PBS+2% fetal bovine serum (FBS) and mixed gently. The diluted samples were centrifuged 20 minutes at 1200 X g on top of lymphocytes separation medium. The enriched cells were then collected from the density medium at plasma interface and washed twice with PBS+2% FBS. Cells were subsequently resuspended in RPMI 1640 medium (Lonza) supplemented with 5% FBS, 50 mM HEPES pH 7.4, glutamine, penicillin, streptomycin and fungizone (complete medium), counted with a Moxi Z mini automated cell counter (ORFLO, MXZ000). Cells suspension was adjusted at 7x10⁶ cells/ml and equilibrated for 1.5 h at 37°C in a 5% CO2 humidified atmosphere.

### PLA2G1B bioassay on CD4 T cells and labelling of specific proteins for optical microscopy

Active human PLA2G1B (75 nM at the end on CD4 T cells) was pretreated or not in PBS BSA 1% for 25 min at room temperature followed by 5 min at 37°C with mAb1 antibody or its control IgG2a isotype: clone eBM2a, eBioscience, 16-4724-85, lot 4291366, at 100 µg/ml at the end. Equilibrated purified CD4 T-cells were loaded (3.5x10⁵cells/50 µL in complete medium) on poly-L-Lysine-coated (Sigma, P8920) round coverslips (14 mm diameter, Marienfeld) in 24-well polystyrene plates at 37°C in a thermo-regulated water, mixed with 50 µL of a suspension containing active human PLA2G1B pretreated or not with antibodies and incubated for 30 min. Cells were then activated for 15 min with 2 nM recombinant glycosylated human IL-7 (Accrobio System, IL7-H4219, lot 359-26Q-8). Cells supernatant was removed and cells were fixed by addition of 500 µL of a 4% paraformaldehyde solution in PBS (Fisher, PFA 32% Electron Microscopy Science, 15714) for 15 min at 37 °C and then permeabilized for 20 min in 500 µL of ice-cold 90%methanol/water solution. Cells were then rehydrated for 15 min in PBS supplemented with 5% fetal bovine serum (FBS) at room temperature and then labelled. Slides were labelled with primary antibodies (1/120) in 60µL of PBS 5% FBS for 1 h, washed in PBS buffer 15 times, 5 times in PBS/FBS buffer and then stained with secondary antibodies (1/300) for 1 h. Slides were washed 5 times in PBS 5% FBS buffer, rinsed 15 times in PBS and then mounted in fresh Prolong Gold Antifade (ThermoFisher Scientific, P36930) mounting medium for confocal microscopy. The primary antibodies used consisted of rabbit anti-pSTAT5 (pY694, 9359, Cell Signaling), goat anti-CD4 (R&D/Novus, AF-379-NA) and secondary antibodies were Donkey anti-goat IgG-AF488 (Invitrogen, A-11055) and Donkey anti-rabbit IgG-AF555 (Invitrogen, A-31572).

### Confocal Microscopy

Images were acquired above the diffraction limit on an inverted laser scanning confocal microscope (LSM700, Zeiss), with an oil-immersion plan-Apochromat 63x/1.4 NA objective lens (Zeiss) for PFA-fixed cells. Images were acquired and analyzed with the ZEN software (Zeiss).

### RESULTS AND DISCUSSION

### Generation of anti-PLA2G1B antibodies that inhibit specifically active PLA2G1B.

Monoclonal antibodies were produced by immunizing mice with recombinant human active PLA2G1B. Immunized mice serums and hybridoma surpernatants were screened by ELISA to identify antibodies specific of active PLA2G1B relatively to other PLA2: PLA2GIIA, PLA2GIID and PLA2GX. Then hybridoma supernatants and antibodies purified from supernatants were screened for the ability to inhibit PLA2G1B enzymatic activity by incubating radiolabeled membranes with recombinant human active PLA2G1B in the presence of increasing amounts of purified monoclonal antibodies. Selected hybridoma were cloned and purified antibodies were re-screened for specificity for PLA2G1B relatively to other PLA2 (PLA2GIIA, PLA2GIID and PLA2GX), inhibitory activity on active human PLA2G1B and specificity for binding to active PLA2G1B relatively to proPLA2G1B by an ELISA assay. Results of PLA2G1B activity inhibition were expressed as percentage of inhibition of enzymatic activity observed with the highest concentration of Ab tested. 22C6 and mAb1 are more specific of active PLA2G1B than proPLA2G1B in ELISA assays, and they are both strong inhibitors of PLA2G1B enzymatic activity with respectively 97% and 100% of inhibition of PLA2G1B activity. The EC50 value for mAb1 antibody binding to active PLA2G1B, as determined by ELISA, was 22 nM.

### Identification of anti-PLA2G1B mAbs that inhibit human PLA2G1B enzymatic activity and label cynomolgus macaque PLA2G1B.

To identify an anti-PLA2G1B mAb that binds to active macaque PLA2G1B we studied binding to cynomolgus macaque pancreas of mAb1 and 22C6 antibodies, as well as six others mAbs (34G3, 23C7, 23F10, 24B2, 28A1, 28A3) that inhibit human PLA2G1B activity (previously described by inventors in the international application WO2019/166664). We tested the binding of all these mAbs at high concentrations on cynomolgus macaque tissues using pancreas as positive tissue and muscle as negative tissue to check for the absence of non-specific labelling. As shown in figure 1, all antibodies were labelling cynomolgus macaque pancreas. Previously, we observed in human pancreas that active PLA2G1B was only present in the exocrine part of the pancreas, proPLA2G1B was expressed in the endocrine part, and human muscle was negative (Pothlichet et al., 2020). We thus postulated that in cynomolgus macaque pancreas active PLA2G1B would also be present in the exocrine part. 23C7 and 28A1 labelling were homogenous on endocrine and exocrine pancreas so they did not seem to be specific of active PLA2G1B under these experimental conditions. 34G3, 23F10, 24B2, 28A3, 22C6 and mAb1 labelling were higher in exocrine pancreas than in endocrine pancreas which shows a higher specificity for active macaque PLA2G1B than proPLA2G1B. 22C6 resulted in lower non-specific labelling on muscle than 34G3, 23F10, 24B2, 28A3 and mAb1 did not result in non-specific labelling of muscle. Thus, we focused our interest on 22C6 and mAb1, two antibodies that differentially labelled exocrine and endocrine part of the pancreas with low or no non-specific labelling of muscle. In this experiment, we used high concentration of antibodies to increase the chance to observe a signal on pancreas that may also result in non-specific labelling on muscle. Then we further studied the labelling conditions of cynomolgus pancreas relatively to cynomolgus muscle for mAb1 and 22C6 to determine conditions specific of active macaque PLA2G1B.

### mAb1 and 22C6 antibodies primarily label the exocrine part of cynomolgus macaque pancreas.

To determine concentrations of mAb1 and 22C6 that specifically label exocrine part of cynomolgus macaque pancreas, cynomolgus macaque pancreas (positive tissue) and muscle (negative tissue) were labelled with different amount of mAb1 or 22C6 antibodies for several times. We found that with 0.5 to 0.1 µg/ml of mAb1 and 0.5 to 0.2 µg/ml of 22C6 we could detect primarily PLA2G1B in the exocrine part of the cynomolgus macaque pancreas (Figure 2). Based on previous observation on human tissues this pattern is due to active PLA2G1B labelling. mAb1 and 22C6 also very slightly labelled endocrine pancreas that could be due to cross-reactivity with proPLA2G1B or slight active PLA2G1B protein level in cynomolgus macaque endocrine pancreas. 22C6 and mAb1 did not label muscle. Uniquely some non-specific binding on interstitial space were observed with 22C6 and mAb1 as well as control isotype antibodies on muscle. These results show that 22C6 and mAb1, preferably at 0.2 µg/ml, could be specific of macaque active PLA2G1B.

### mAb1 and 22C6 antibodies preferentially label the exocrine part of human pancreas.

To determine concentrations of mAb1 and 22C6 that specifically label human active PLA2G1B in tissues, frozen human pancreas (positive tissue) and muscle (negative tissue) were labelled with different amounts of mAb1 or 22C6 antibodies. We found that mAb1 and 22C6 preferentially label human exocrine pancreas at 0.2 and 0.1 µg/ml (Figure 3). No signal was observed on muscle under these experimental conditions. This shows that these conditions preferentially detect human active PLA2G1B in tissues with the highest specificity for active PLA2G1B at about 0.1 µg/ml.

### mAb1 and 22C6 antibodies specifically label the human active PLA2G1B in CHO cells overexpressing active PLA2G1B.

To check the specificity for active PLA2G1B of mAb1 and 22C6 concentrations that specifically label exocrine part of cynomolgus and human pancreas, these labelling conditions were tested on CHO cells that express either human active PLA2G1B, or human proPLA2G1B or not transfected. mAb1 and 22C6 preferentially label active PLA2G1B than proPLA2G1B with the highest difference at 0.2 and 0.1 µg/ml (Figure 4). mAb1 does not label untransfected cells but some labelling is observed with 22C6 on untransfected cells which shows that mAb 1 results in a more specific labelling than 22C6. Some cross-reactivity with proPLA2G1B is observed even at 0.1 µg/ml of mAb1 or 22C6. Taken together these results support our hypothesis that these experimental conditions preferentially labelled human active PLA2G1B and show that 22C6 and mAb 1 bind preferentially to active human and macaque PLA2G1B.

Altogether labelling tests with mAb1 and 22C6 indicate that to preferentially detect active PLA2G1B these antibodies should be used at about 0.2 µg/ml or more (preferably at 0.2 µg/ml) on cynomolgus tissues and at about 0.1 µg/ml or more (preferably at 0.1 µg/ml) on human tissues.

### mAb1 and 22C6 antibodies bind with high affinity to active human and macaque PLA2G1B.

To study mAb1 and 22C6 binding to human and cynomolgus macaque active PLA2G1B and proPLA2G1B, recombinant proteins were produced and binding of 22C6 and mAb1 antibodies to these proteins were studied by surface plasmon resonance technology on a BIAcore biosensor (Table 1). The half-life of the 22C6 or mAb1 antibody/active hPLA2G1B complex is higher than 38 h and almost 10 h respectively (t ½ >2300 and = 590 min, respectively) meaning that these complexes are quasi-irreversible. Notably, 22C6 and mAb1 affinity for active hPLA2G1B is extremely high with Kd ≤ 1 pM and Kd ≤ 2 pM respectively. 22C6 mAb affinity is more than 3000-fold higher for hPLA2G1B than hproPLA2G1B. 22C6 and mAb1 affinity for active macPLA2G1B is also very high with Kd of 53 pM and 62 pM respectively. 22C6 antibody affinity is 1425-Fold higher for macPLA2G1B than macproPLA2G1B. mAb1 antibody affinity is >1295-fold higher for hPLA2G1B than hproPLA2G1B. mAb1 antibody affinity is 1095-Fold higher for macPLA2G1B than macproPLA2G1B. Altogether these results show that both antibodies are highly more specific of active PLA2G1B than proPLA2G1B in both human and macaque.

**Table 1: 22C6 and mAb1 antibodies affinity for human and macaque PLA2G1B and proPLA2G1B: The affinity constant (Kd) of the human and macaque proPLA2G1B (hproPLA2G1B and macproPLA2G1B, respectively) and active PLA2G1B (hPLA2G1B and macPLA2G1B, respectively) binding to 22C6 and mAb1 antibodies were determined by surface kinetics on a real-time biosensor surface plasmon resonance assay (BIAcore^{™}). The antibody was captured on an anti-mouse IgG surface and exposed to various concentrations of recombinant proPLA2G1B and active PLA2G1B proteins. Kinetic analysis was performed to obtain the association (Kon) and dissociation (Koff) rate constants. Kon, Koff, t ½ and Kd values are presented.**

| mAb | Antigen | kₒₙ (10⁴ M⁻¹.s⁻¹) | k_{off} (10⁻⁴ s⁻¹) | t_{1/2} (min) | K_{d} (nM) |
|---|---|---|---|---|---|
| 22C6 | hPLA2G1B | > 1000 ⁽¹⁾ | < 0.1 ⁽¹⁾ | > 2300 | ≤ 1pM⁽¹⁾ |
| 22C6 | hproPLA2G1B | 59.5 ± 10.7 | 17.7 ± 4.3 | 6.5 | 3.0 ± 0.4 |
| 22C6 | macPLA2G1B | 350 ± 8 | 1.79 ± 0.06 | 64.5 | 0.053 ± 0.009 |
| 22C6 | macproPLA2G1B | 32.4 ± 2.1 | 247 ± 25 | 0.47 | 75.5 ± 7.9 |
| mAb1 | hPLA2G1B | > 1000 ⁽¹⁾ | 0.19 ± 0.01 | 590 | ≤ 2pM⁽¹⁾ |
| mAb1 | hproPLA2G1B | 51.2 ± 6.8 | 13.2 ± 2.3 | 8.7 | 2.59 ± 0.04 |
| mAb1 | macPLA2G1B | 310 ± 19 | 1.79 ± 0.02 | 64.5 | 0.062 ± 0.001 |
| mAb1 | macproPLA2G1B | 27.5 ± 3.0 | 184 ± 10 | 0.63 | 67.9 ± 8.7 |

| | | | | | |
|---|---|---|---|---|---|
| (1) in the table means kinetic constants exceed limits that can be reliably measured by the instrument. | | | | | |

### Sequencing of 22C6 and mAb1 variable regions confirmed that no equivalent sequences have been previously described in database before Inventors studies.

To confirm the high Kd value measured with mAb 1 and 22C6 antibodies we decided to produce 22C6 and mAb1 Fabs. Therefore, we sequenced variable light (VL) and heavy (VL) chain regions (Figure 5 and 6). The 22C6 variable domain sequences were previously described by inventors in the international application WO2019/166664 but here we added information on prediction of functional subdomains and model of 3D structure (Figure 5).

In the present application, we provide, for the first time, sequences of mAb1 variable regions (Figure 6).

These sequences confirmed that both antibodies light chains are kappa isotype (SEQ ID NO: 9-12, Table 2 and Fig. 5A for 22C6; SEQ ID NO: 1-4, Table 4 and Fig. 6A for mAb1) and heavy chains are IgG2a isotypes (SEQ ID NO: 13-16, Table 3 and Fig. 5B for 22C6; SEQ ID NO: 5-8, Table 5 and Fig. 6B for mAb1) and allow the prediction of their functional subdomains FR1-FR4 and CDR1-3 and to establish a model of representation of the 3D structure (IMGT Colliers de Perles according to Pommié et al., 2004) of the VL (Figures 5A and 6A) and VH (Figures 5B and 6B). The analysis of VL (Table 2 and 4) and VH (Table 3 and 5) sequence identity with mouse germinal sequences demonstrate that 22C6 and mAb1 sequences are unique with no equivalent sequence previously described in database (Uniprot databank-UniprotkB).

**Table 2: Comparison of 22C6 VL with mouse germinal sequences (http://www.imgt.org/IMGT_vquest/share/textes/). 22C6 VL is a rearranged and productive IGK sequence, Kappa isotype, with no equivalent in database (Uniprot databank-UniprotKB)**

| **22C6-3A6-VL** | **Rearranged and productive IGK sequence** | | |
|---|---|---|---|
| | Mouse germinal sequences with the highest identity | Percentage of identity between 22C6-3A6 antibody and mouse germinal sequences with the highest identity | |
| V-GENE (*allele) | IGKV4-91*01 F | 95.39% | |
| J-GENE (*allele) | IGKJ5*01 F | 100% | |
| Lengths of FR, CDR and junction sequences (AA) | [26.17.36.10] | [7.3.9] | CHQGNSLPLTF |

**Table 3: Comparison of 22C6 VH with mouse germinal sequences (http://www.imgt.org/IMGT_vquest/share/textes/). 22C6 VH is a rearranged and productive IGH sequence, IgG2a isotype, with no equivalent in database (Uniprot databank-UniprotKB)**

| **22C6-3A6-VH** | **Rearraged and productive IGH sequence** | | |
|---|---|---|---|
| | Mouse germinal sequences with the highest identity | Percentage of identity between 22C6-3A6 antibody and mouse germinal sequences with the highest identity | |
| V-GENE (*allele) | IGHV3-4*01 F | 89.69% | |
| J-GENE (*allele) | IGHJ2*01 | 89.58% | |
| D-GENE (*allele) | IGHD2-3*01 | D-REGION (ORF3) | |
| Lengths of FR, CDR and junction sequences (AA) | [25.17.38.11] | [10.7.13] | CARDSTDGDFYFEKW |

**Table 4: Comparison of mAb1 VL with mouse germinal sequences (http://www.imgt.org/IMGT_vquest/share/textes/). mAb1 VL is a rearranged and productive IGK sequence, Kappa isotype, with no equivalent in database (Uniprot databank-UniprotKB).**

| **mAb1-VL** | **Rearranged and productive IGK sequence** | | |
|---|---|---|---|
| | Mouse germinal sequences with the highest identity | Percentage of identity between mAb1 antibody and mouse germinal sequences with the highest identity | |
| **V-GENE (*allele)** | IGKV3-5*01 F | 96.91% | |
| **J-GENE (*allele)** | IGKJ2*01 F | 97.44% | |
| **Lengths of FR, CDR and junction sequences (AA)** | [26.17.36.10] | [10.3.9] | CQQSSRDLFTF |

**Table 5: Comparison of mAb1 VH with mouse germinal sequences (http://www.imgt.org/IMGT_vquest/share/textes/). mAb1 VH is a rearranged and productive IGH sequence, IgG2a isotype, with no equivalent in database (Uniprot databank-UniprotKB).**

| **mAb1-VH** | **Rearranged and productive IGH sequence** | | |
|---|---|---|---|
| | Mouse germinal sequences with the highest identity | Percentage of identity between mAb1 antibody and mouse germinal sequences with the highest identity | |
| **V-GENE (*allèle)** | IGHV3-4*01 F | 90.72 % | |
| **J-GENE (*allèle)** | IGHJ2*01 | 87.50% | |
| **D-GENE (*allèle)** | IGHD2-3*01 | D-REGION (ORF3) | |
| **Lengths of FR, CDR and junction sequences (AA)** | [25.17.38.11] | [10.7.13] | CARDSTDGDFYFEKW |

### Fab also binds with high affinity to active human and macaque PLA2G1B.

Based on the sequence of variable regions of mAb1, recombinant Fab was produced in CHO cells. mAb1 Fab binding to human and cynomolgus active and proPLA2G1B were then studied. mAb1 Fab was very efficiently binding to both human and cynomolgus macaque active PLA2G1B and with a much higher affinity for active PLA2G1B than proPLA2G1B in both species (Table 6). Half-life of the mAb1 Fab/hPLA2G1B complex is almost 4h (t ½ = 226 min) indicating that mAb1 Fab/hPLA2G1B complex is quasi-irreversible. mAb1 Fab affinity for hPLA2G1B is extremely high with a Kd of 6 pM. mAb1 Fab affinity is 273-fold higher for hPLA2G1B than hproPLA2G1B. mAb1 is also high for macPLA2G1B with a Kd of 34pM. mAb1 Fab affinity is 1409-Fold higher for macPLA2G1B than macproPLA2G1B. These results with mAb1 Fab confirmed the high affinity of mAb1 antibody for active PLA2G1B in both species observed with mAb1 antibody.

**Table 6: mAb1 Fab affinity for human and macaque PLA2G1B and proPLA2G1B. The affinity constant (Kd) of the human and macaque proPLA2G1B (hproPLA2G1B and macproPLA2G1B, respectively) and active PLA2G1B (hPLA2G1B and macPLA2G1B, respectively) binding to mAb1 Fab were determined by surface kinetics on a real-time biosensor surface plasmon resonance assay (BIAcore^{™}). The mAb1 Fab was captured on an anti-mouse Fab surface and exposed to various concentrations of recombinant proPLA2G1B and active PLA2G1B proteins. Kinetic analysis was performed to obtain the association (Kon) and dissociation (Koff) rate constants. Kon, Koff, t ½ and Kd values are presented.**

| Fab | Antigen | kₒₙ (10⁴ M⁻¹ .s⁻¹) | k_{off} (10⁻⁴ s⁻¹) | t_{1/2} (min) | K_{d} (nM) |
|---|---|---|---|---|---|
| mAb1 | hPLA2G1B | 815 ± 36 | 0.51 ± 0.14 | 226 | 0.006 ± 0.001 |
| mAb1 | hproPLA2G1B | 76.1 ± 13.7 | 10.4 ± 0.6 | 11 | 1.64 ± 0.22 |
| mAb1 | macPLA2G1B | 403.6 ± 21.3 | 1.33 ± 0.03 | 87 | 0.034 ± 0.005 |
| mAb1 | macproPLA2G1B | 57.2 ± 2.87 | 273 ± 15 | 0.4 | 47.9 ± 0.3 |

### 22C6 and mAb1 antibodies are potent inhibitors of both human and cynomolgus macaque PLA2G1B enzymatic activity.

To study the effect of 22C6 and mAb1 antibodies on active human and macaque PLA2G1B enzymatic activity recombinant PLA2G1B were pre-incubated with several concentrations of antibodies and then enzymatic activity was evaluated in the tritiated oleic acid release assay using radiolabelled *E. Coli* membranes (Figure 7). Both antibodies are potent inhibitors of active PLA2G1B from human and macaque. The 22C6 IC50 was 58 pM on human PLA2G1B and 430 pM on macPLA2G1B. The mAb1 IC50 was 78 pM on human PLA2G1B and 590 pM on macaque PLA2G1B.

These results indicate that 22C6 and mAb1 could be very potent antibodies to block PLA2G1B activity in human and macaque. Thus, they are therapeutic candidates to block adverse effect of PLA2G1B in HIV patients (Pothlichet et al., 2020) and could also be used to control SIV or SHIV infection and adverse effect in cynomolgus macaques.

### mAb1 antibody strongly blocks PLA2G1B inhibitory activity on primary human CD4 T cells.

To see if mAb1 can inhibit PLA2G1B in human CD4 T cells, PLA2G1B protein was preincubated or not with mAb1 antibody or control isotype and then antibody-pretreated PLA2G1B was added on human primary CD4 T cells isolated from healthy donors (n= 2 to 3 experiments, 1 donor/ experiment). Notably mAb1 almost abrogates PLA2G1B inhibitory activity on CD4 T cells relatively to control isotype-treated PLA2G1B or untreated PLA2G1B. These results clearly show that mAb1 can block PLA2G1B activity on human CD4 T cells. We have also showed previously that this PLA2G1B activity is involved in CD4 T cells anergy and lymphopenia in HIV-infected patients (Pothlichet et al., 2020).

Altogether, the high affinity of 22C6 and mAb1 and their inhibitory activity on human and macaque PLA2G1B allowed us to propose that injection of these antibodies in human or macaque could be a new approach to restore CD4 T-cell function and may impair the onset of immunodeficiency or an associated disease, in particular, in HIV-, SIV- or SHIV-infected subj ects.

### REFERENCES

Barre-Sinoussi F, Ross AL, Delfraissy JF. Past, present and future: 30 years of HIV research. Nat Rev Microbiol. 2013 Dec;11(12):877-83.
Eskola, J.U., Nevalainen, T.J., Aho, H.J., 1983. Purification and Characterization of Human Pancreatic Phospholipase A2. 29(10), pp.1772-1776.
Kitagawa, M. et al., 1991. The diagnostic value of serum pancreatic phospholipase A2 (PLA2) in pancreatic diseases. Gastroenterologia Japonica, 26(1), pp.62-68.
Lambeau G, Gelb MH. Biochemistry and physiology of mammalian secreted phospholipases A2. Annu Rev Biochem. 2008; 77:495-520.
Müller-Trutwin MC, Corbet S, Tavares MD, Hervé VM, Nerrienet E, Georges-Courbot MC, Saurin W, Sonigo P, Barre-Sinoussi F. The evolutionary rate of nonpathogenic simian immunodeficiency virus (SIVagm) is in agreement with a rapid and continuous replication in vivo. Virology. 1996 Sep 1;223(1):89-102.
Nakano, T. et al., 1994. Plasmin converts pro-form of group I phospholipase A2 into receptor binding, active forms. Biochemical and biophysical research communications, 198(1), pp. 10-15.
Nishijima, J. et al., 1983. Purification and characterization of human pancreatic phospholipase A2 and development of a radioimmunoassay. Journal of biochemistry, 94(1), pp. 137-147.
Nishimura Y, Gautam R, Chun TW, Sadjadpour R, Foulds KE, Shingai M, Klein F, Gazumyan A, Golijanin J, Donaldson M, Donau OK, Plishka RJ, Buckler-White A, Seaman MS, Lifson JD, Koup RA, Fauci AS, Nussenzweig MC, Martin MA. Early antibody therapy can induce long-lasting immunity to SHIV. Nature. 2017 Mar 23;543(7646):559-563.
Pommié C, Levadoux S, Sabatier R, Lefranc G, Lefranc MP. et al. IMGT standardized criteria for statistical analysis of immunoglobulin V-REGION amino acid properties. Journal of Molecular Recognition. 2004:17(1), 17-32.
Ponte R, Mehraj V, Ghali P, Couëdel-Courteille A, Cheynier R, Routy JP. Reversing Gut Damage in HIV Infection: Using Non-Human Primate Models to Instruct Clinical Research. EBioMedicine. 2016 Jan 26; 4:40-9.
Pothlichet et al., PLA2G1B is involved in CD4 anergy and CD4 lymphopenia in HIV-infected patients. J Clin Invest. 2020 Jun 1;130(6):2872-2887
Rey-Cuillé MA, Berthier JL, Bomsel-Demontoy MC, Chaduc Y, Montagnier L, Hovanessian AG, Chakrabarti LA. Simian immunodeficiency virus replicates to high levels in sooty mangabeys without inducing disease. J Virol. 1998 May;72(5):3872-86.
Rouault M, Le Calvez C, Boilard E, Surrel F, Singer A, Ghomashchi F, Bezzine S, Scarzello S, Bollinger J, Gelb MH, Lambeau G. Recombinant production and properties of binding of the full set of mouse secreted phospholipases A2 to the mouse M-type receptor. Biochemistry. 2007 Feb 13; 46(6): 1647-62.
Singer AG, Ghomashchi F, Le Calvez C, Bollinger J, Bezzine S, Rouault M, Sadilek M, Nguyen E, Lazdunski M, Lambeau G, Gelb MH. Interfacial kinetic and binding properties of the complete set of human and mouse groups I, II, V, X, and XII secreted phospholipases A2. J Biol Chem. 2002 Dec 13; 277(50):48535-49.
Sodora DL, Allan JS, Apetrei C, Brenchley JM, Douek DC, Else JG, Estes JD, Hahn BH, Hirsch VM, Kaur A, Kirchhoff F, Muller-Trutwin M, Pandrea I, Schmitz JE, Silvestri G. Toward an AIDS vaccine: lessons from natural simian immunodeficiency virus infections of African nonhuman primate hosts. Nat Med. 2009 Aug; 15(8):861-5.
Sternby B., Akerstrom B. Immunoreactive pancreatic colipase, lipase and phospholipase A2 in human plasma and urine from healthy individuals. Biochim. Biophys Acta 1984; 789, 164-169.
Veazey RS, DeMaria M, Chalifoux LV, Shvetz DE, Pauley DR, Knight HL, Rosenzweig M, Johnson RP, Desrosiers RC, Lackner AA. Gastrointestinal tract as a major site of CD4+ T cell depletion and viral replication in SIV infection. Science. 1998 Apr 17;280(5362):427-31.

### SEQUENCES

**SEQ ID NO: 1** amino acid sequence of mAb1 variable light chain region (mAb1-VL) 
DIVLTQSPASLAVSLGQRATISCRASESVDNYGISFMHWYQQKPGQPPKLLIYRASNL
ESGIPARFSGSGSRTDFTLTINPVETDDVATYYCQQSSRDLFTFGGGTKLEIK
**SEQ ID NO: 2** amino acid sequence of mAb1 constant light chain region Ck (Mus musculus IGKC*03) 
RADAAPTVSIFPPSSEQLTSGGASVVCFLNNFYPRDINVKWKIDGSERQNGVLNSWTD
QDSKDSTYSMSSTLTLTKDEYERHNSYTCEATHKTSTSPIVKSFNRNEC
**SEQ ID NO: 3** nucleotide sequence of mAb1 variable light chain region (mAb1-VL) 
GACATTGTGCTGACCCAATCTCCAGCTTCTTTGGCTGTGTCTCTAGGGCAGAGGG
CCACCATCTCCTGCAGAGCCAGCGAAAGTGTTGATAATTATGGCATTAGTTTTAT
GCACTGGTACCAGCAGAAACCGGGACAGCCACCCAAACTCCTCATCTATCGTGCA
TCCAACCTAGAATCTGGGATCCCTGCCAGGTTCAGTGGCAGTGGGTCTAGGACAG
ACTTCACCCTCACCATTAATCCTGTGGAGACTGATGATGTTGCAACCTATTACTGT
CAGCAAAGTAGTAGGGATCTGTTCACGTTCGGAGGGGGGACCAAGCTGGAAATA
AAA
**SEQ ID NO: 4** nucleotide sequence of mAb1 constant light chain region Ck (Mus musculus IGKC*03) 
CGGGCTGATGCTGCACCAACTGTATCCATCTTCCCACCATCCAGTGAGCAGTTAA
CATCTGGAGGTGCCTCAGTCGTGTGCTTCTTGAACAACTTCTACCCCAGAGACAT
CAATGTCAAGTGGAAGATTGATGGCAGTGAACGACAAAATGGTGTCCTGAACAG
TTGGACTGATCAGGACAGCAAAGACAGCACCTACAGCATGAGCAGCACCCTCAC
ATTGACCAAGGACGAGTATGAACGACATAACAGCTATACCTGTGAGGCCACTCA
CAAGACATCAACTTCACCCATCGTCAAGAGCTTCAACAGGAATGAGTGT
**SEQ ID NO: 5** amino acid sequence of mAb1 variable heavy chain region (mAb1-VH) 
DVQLQESGPGLVKPSQTVSLNCTVSGHSITDDNYYWSWIRLVSGSKLEWIGYITSSGN
TDNNPSLKSRISVTRDISKNQFFLHLSSVTAEDSATYYCARDSTDGDFYFEKWGRGTT
LTVSS
**SEQ ID NO: 6** amino acid sequence of mAb1 constant heavy chain region (Mus musculus IGHG2a*01) 
AKTTAPSVYPLAPVCGDTSGSSVTLGCLVKGYFPEPVTLTWNSGSLSSGVHTFPAVL
QSDLYTLSSSVTVTSSTWPSQSITCNVAHPASSTKVDKKIEPRGPTIKPCPPCKCPAPNL
LGGPSVFIFPPKIKDVLMISLSPIVTCVVVDVSEDDPDVQISWFVNNVEVHTAQTQTH
REDYNSTLRVVSALPIQHQDWMSGKEFKCKVNNKDLPAPIERTISKPKGSVRAPQVY
VLPPPEEEMTKKQVTLTCMVTDFMPEDIYVEWTNNGKTELNYKNTEPVLDSDGSYF
MYSKLRVEKKNWVERNSYSCSVVHEGLHNHHTTKSFSRTPGK
**SEQ ID NO: 7** nucleotide sequence of mAb1 variable heavy chain region (mAb1-VH) 
GATGTACAGCTTCAGGAGTCAGGACCTGGCCTGGTGAAGCCTTCTCAGACAGTGT
CCCTCAACTGCACTGTCTCTGGCCACTCTATCACTGATGATAATTACTATTGGAGC
TGGATCCGGCTGGTTTCAGGAAGCAAACTGGAGTGGATAGGGTACATAACCTCC
AGTGGTAACACTGACAACAATCCTTCTCTCAAAAGTCGCATCTCCGTCACCCGGG
ACATTTCCAAGAACCAGTTCTTCCTGCACTTGAGCTCTGTGACTGCTGAAGATTCA
GCCACATATTACTGTGCAAGAGATTCAACTGATGGTGACTTTTACTTTGAAAAT
GGGGCCGAGGCACCACTCTCACAGTCTCCTCA
**SEQ ID NO: 8** nucleotide sequence of mAb1 constant heavy chain region (Mus musculus IGHG2a*01) 
GCCAAAACAACAGCCCCATCGGTCTATCCACTGGCCCCTGTGTGTGGAGATACAA
GTGGCTCCTCGGTGACTCTAGGATGCCTGGTCAAGGGTTATTTCCCTGAGCCAGT
GACCTTGACCTGGAACTCTGGATCCCTGTCCAGTGGTGTGCACACCTTCCCAGCT
GTCCTGCAGTCTGACCTCTACACCCTCAGCAGCTCAGTGACTGTAACCTCGAGCA
CCTGGCCCAGCCAGTCCATCACCTGCAATGTGGCCCACCCGGCAAGCAGCACCAA
GGTGGACAAGAAAATTGAGCCCAGAGGGCCCACAATCAAGCCCTGTCCTCCATG
CAAATGCCCAGCACCTAACCTCTTGGGTGGACCATCCGTCTTCATCTTCCCTCCAA
AGATCAAGGATGTACTCATGATCTCCCTGAGCCCCATAGTCACATGTGTGGTGGT
GGATGTGAGCGAGGATGACCCAGATGTCCAGATCAGCTGGTTTGTGAACAACGT
GGAAGTACACACAGCTCAGACACAAACCCATAGAGAGGATTACAACAGTACTCT
CCGGGTGGTCAGTGCCCTCCCCATCCAGCACCAGGACTGGATGAGTGGCAAGGA
GTTCAAATGCAAGGTCAACAACAAAGACCTCCCAGCGCCCATCGAGAGAACCAT
CTCAAAACCCAAAGGGTCAGTAAGAGCTCCACAGGTATATGTCTTGCCTCCACCA
GAAGAAGAGATGACTAAGAAACAGGTCACTCTGACCTGCATGGTCACAGACTTC
ATGCCTGAAGACATTTACGTGGAGTGGACCAACAACGGGAAAACAGAGCTAAAC
TACAAGAACACTGAACCAGTCCTGGACTCTGATGGTTCTTACTTCATGTACAGCA
AGCTGAGAGTGGAAAAGAAGAACTGGGTGGAAAGAAATAGCTACTCCTGTTCAG
TGGTCCACGAGGGTCTGCACAATCACCACACGACTAAGAGCTTCTCCCGGACTCC
GGGTAAA
**SEQ ID NO: 9** amino acid sequence of 22C6 variable light chain domain (22C6-VL) 
EIVLTQSPTTMAASPGEKITITCSVSSSISPNFLHWYQQKPGFSPKLLIFRTSNLASGVPT
RFSGSGSGTSYSLTIGTMEAEDVATYYCHQGNSLPLTFGAGTKLELK
**SEQ ID NO: 10** partial amino acid sequence of 22C6 constant light chain domain Ck (Mus musculus IGKC*01) RADAAPTVSIFPP
**SEQ ID NO: 11** nucleotide sequence of 22C6 variable light chain domain (22C6-VL) 
GAAATTGTTCTCACCCAGTCTCCAACCACCATGGCTGCATCTCCCGGGGAGAAGA
TCACTATCACCTGCAGCGTCAGCTCAAGTATAAGTCCCAATTTCTTGCATTGGTAT
CAGCAGAAGCCAGGATTCTCCCCGAAACTCTTGATTTTTAGGACATCCAATCTGG
CTTCTGGAGTCCCAACTCGCTTCAGTGGCAGTGGGTCTGGGACCTCTTACTCTCTC
 
ACAATTGGCACCATGGAGGCTGAAGACGTTGCCACTTATTACTGTCACCAGGGTA
ATAGTTTACCGCTCACGTTCGGTGCTGGGACCAAGCTGGAGCTGAAA
**SEQ ID NO: 12** partial nucleotide sequence of 22C6 constant light chain domain Ck (Mus musculus IGKC*01)
   CGGGCTGATGCTGCACCAACTGTATCCATCTTCCCACCATCC
**SEQ ID NO: 13** amino acid sequence of 22C6 variable heavy chain domain (22C6-VH) 
EVQLQESGPGLVKPSQTVSLNCTVTGHSITDDNYYWSWIRLVSGSKLEWIGYITSAG
NTDNNPSLKSRISITRDISKNQFFLHLSSVTAEDTATYYCARDSTDGDFYFEKWGQGT
TLTVSS
**SEQ ID NO: 14** partial amino acid sequence of 22C6 constant heavy chain domain CH1 (Mus musculus IGHG2a*01)
   AKTTAPSVYPLAPVCGDTSGSSVTLGCLVK
**SEQ ID NO: 15** nucleotide sequence of 22C6 variable heavy chain domain (22C6-VH) 
GAGGTGCAGCTGCAGGAGTCAGGACCTGGCCTGGTGAAGCCTTCTCAGACAGTG
TCCCTCAACTGCACTGTCACTGGCCACTCTATCACTGATGATAATTACTATTGGAG
CTGGATCCGGCTGGTTTCAGGAAGCAAACTGGAGTGGATAGGGTACATAACCTCC
GCTGGTAACACTGACAACAATCCTTCTCTCAAAAGTCGCATCTCCATCACCCGGG
ACATTTCCAAGAACCAATTCTTCCTGCACTTGAGCTCTGTGACTGCTGAAGATAC
AGCCACATATTACTGTGCAAGAGATTCAACTGATGGTGACTTTTACTTTGAAAAA
TGGGGCCAAGGCACCACTCTCACAGTCTCCTCA
**SEQ ID NO: 16** partial nucleotide sequence of constant 22C6 heavy chain domain CH1 (Mus musculus IGHG2a*01) 
GCCAAAACAACAGCCCCATCGGTCTATCCACTGGCCCCTGTGTGTGGAGATACAA
GTGGCTCCTCGGTGACTCTAGGATGCCTGGTCAAG
**SEQ ID NO: 17** full nucleotide sequence of mAb1 light chain (mAb1-LC) 
GACATTGTGCTGACCCAATCTCCAGCTTCTTTGGCTGTGTCTCTAGGGCAGAGGG
CCACCATCTCCTGCAGAGCCAGCGAAAGTGTTGATAATTATGGCATTAGTTTTAT
GCACTGGTACCAGCAGAAACCGGGACAGCCACCCAAACTCCTCATCTATCGTGCA
TCCAACCTAGAATCTGGGATCCCTGCCAGGTTCAGTGGCAGTGGGTCTAGGACAG
ACTTCACCCTCACCATTAATCCTGTGGAGACTGATGATGTTGCAACCTATTACTGT
CAGCAAAGTAGTAGGGATCTGTTCACGTTCGGAGGGGGGACCAAGCTGGAAATA
AAACGGGCTGATGCTGCACCAACTGTATCCATCTTCCCACCATCCAGTGAGCAGT
TAACATCTGGAGGTGCCTCAGTCGTGTGCTTCTTGAACAACTTCTACCCCAGAGA
CATCAATGTCAAGTGGAAGATTGATGGCAGTGAACGACAAAATGGTGTCCTGAA
CAGTTGGACTGATCAGGACAGCAAAGACAGCACCTACAGCATGAGCAGCACCCT
CACATTGACCAAGGACGAGTATGAACGACATAACAGCTATACCTGTGAGGCCAC
TCACAAGACATCAACTTCACCCATCGTCAAGAGCTTCAACAGGAATGAGTGT
**SEQ ID NO: 18** full amino acid sequence of mAb1 light chain (mAb1-LC) 
DIVLTQSPASLAVSLGQRATISCRASESVDNYGISFMHWYQQKPGQPPKLLIYRASNL
ESGIPARFSGSGSRTDFTLTINPVETDDVATYYCQQSSRDLFTFGGGTKLEIKRADAAP
TVSIFPPSSEQLTSGGASVVCFLNNFYPRDINVKWKIDGSERQNGVLNSWTDQDSKDS
TYSMSSTLTLTKDEYERHNSYTCEATHKTSTSPIVKSFNRNEC
**SEQ ID NO: 19** full nucleotide sequence of mAb1 heavy chain (mAb1-HC) 
GATGTACAGCTTCAGGAGTCAGGACCTGGCCTGGTGAAGCCTTCTCAGACAGTGT
CCCTCAACTGCACTGTCTCTGGCCACTCTATCACTGATGATAATTACTATTGGAGC
TGGATCCGGCTGGTTTCAGGAAGCAAACTGGAGTGGATAGGGTACATAACCTCC
AGTGGTAACACTGACAACAATCCTTCTCTCAAAAGTCGCATCTCCGTCACCCGGG
ACATTTCCAAGAACCAGTTCTTCCTGCACTTGAGCTCTGTGACTGCTGAAGATTCA
GCCACATATTACTGTGCAAGAGATTCAACTGATGGTGACTTTTACTTTGAAAAT
GGGGCCGAGGCACCACTCTCACAGTCTCCTCAGCCAAAACAACAGCCCCATCGGT
CTATCCACTGGCCCCTGTGTGTGGAGATACAAGTGGCTCCTCGGTGACTCTAGGA
TGCCTGGTCAAGGGTTATTTCCCTGAGCCAGTGACCTTGACCTGGAACTCTGGAT
CCCTGTCCAGTGGTGTGCACACCTTCCCAGCTGTCCTGCAGTCTGACCTCTACACC
CTCAGCAGCTCAGTGACTGTAACCTCGAGCACCTGGCCCAGCCAGTCCATCACCT
GCAATGTGGCCCACCCGGCAAGCAGCACCAAGGTGGACAAGAAAATTGAGCCCA
GAGGGCCCACAATCAAGCCCTGTCCTCCATGCAAATGCCCAGCACCTAACCTCTT
GGGTGGACCATCCGTCTTCATCTTCCCTCCAAAGATCAAGGATGTACTCATGATC
TCCCTGAGCCCCATAGTCACATGTGTGGTGGTGGATGTGAGCGAGGATGACCCAG
ATGTCCAGATCAGCTGGTTTGTGAACAACGTGGAAGTACACACAGCTCAGACAC
AAACCCATAGAGAGGATTACAACAGTACTCTCCGGGTGGTCAGTGCCCTCCCCAT
CCAGCACCAGGACTGGATGAGTGGCAAGGAGTTCAAATGCAAGGTCAACAACAA
AGACCTCCCAGCGCCCATCGAGAGAACCATCTCAAAACCCAAAGGGTCAGTAAG
AGCTCCACAGGTATATGTCTTGCCTCCACCAGAAGAAGAGATGACTAAGAAACA
GGTCACTCTGACCTGCATGGTCACAGACTTCATGCCTGAAGACATTTACGTGGAG
TGGACCAACAACGGGAAAACAGAGCTAAACTACAAGAACACTGAACCAGTCCTG
GACTCTGATGGTTCTTACTTCATGTACAGCAAGCTGAGAGTGGAAAAGAAGAACT
GGGTGGAAAGAAATAGCTACTCCTGTTCAGTGGTCCACGAGGGTCTGCACAATCA
CCACACGACTAAGAGCTTCTCCCGGACTCCGGGTAAA
**SEQ ID NO: 20** full amino acid sequence of mAb1 heavy chain (mAb1-HC) 
DVQLQESGPGLVKPSQTVSLNCTVSGHSITDDNYYWSWIRLVSGSKLEWIGYITSSGN
TDNNPSLKSRISVTRDISKNQFFLHLSSVTAEDSATYYCARDSTDGDFYFEKWGRGTT
LTVSSAKTTAPSVYPLAPVCGDTSGSSVTLGCLVKGYFPEPVTLTWNSGSLSSGVHTF
PAVLQSDLYTLSSSVTVTSSTWPSQSITCNVAHPASSTKVDKKIEPRGPTIKPCPPCKCP
APNLLGGPSVFIFPPKIKDVLMISLSPIVTCVVVDVSEDDPDVQISWFVNNVEVHTAQT
QTHREDYNSTLRVVSALPIQHQDWMSGKEFKCKVNNKDLPAPIERTISKPKGSVRAP
QVYVLPPPEEEMTKKQVTLTCMVTDFMPEDIYVEWTNNGKTELNYKNTEPVLDSDG
SYFMYSKLRVEKKNWVERNSYSCSVVHEGLHNHHTTKSFSRTPGK
**SEQ ID NO: 21** amino acid sequence of CDR1 of mAb1-VL
   ESVDNYGISF
**SEQ ID NO: 22** amino acid sequence of CDR2 of mAb1-VL
   RAS
**SEQ ID NO: 23** amino acid sequence of CDR3 of mAb1-VL
   QQSSRDLFT
**SEQ ID NO: 24** amino acid sequence of CDR1 of mAb1-VH
   GHSITDDNYY
**SEQ ID NO: 25** amino acid sequence of CDR2 of mAb1-VH
   ITSSGNT
**SEQ ID NO: 26** amino acid sequence of CDR3 of mAb1-VH
   ARDSTDGDFYFEK
**SEQ ID NO: 27** amino acid sequence of CDR1 of 22C6-VL
   SSISPNF
**SEQ ID NO: 28** amino acid sequence of CDR2 of 22C6-VL
   RTS
**SEQ ID NO: 29** amino acid sequence of CDR3 of 22C6-VL
   HQGNSLPLT
**SEQ ID NO: 30** amino acid sequence of CDR1 of 22C6-VH
   GHSITDDNYY
**SEQ ID NO: 31** amino acid sequence of CDR2 of 22C6-VH
   ITSAGNT
**SEQ ID NO: 32** amino acid sequence of CDR3 of 22C6-VH
   ARDSTDGDFYFEK
**SEQ ID NO: 33** amino acid sequence of constant CH1 domain of mAb1-VH 
AKTTAPSVYPLAPVCGDTSGSSVTLGCLVKGYFPEPVTLTWNSGSLSSGVHTFPAVL
QSDLYTLSSSVTVTSSTWPSQSITCNVAHPASSTKVDKKI
**SEQ ID NO: 34** amino acid sequence of constant CH2 domain of mAb1-VH 
APNLLGGPSVFIFPPKIKDVLMISLSPIVTCVVVDVSEDDPDVQISWFVNNVEVHTAQT
QTHREDYNSTLRVVSALPIQHQDWMSGKEFKCKVNNKDLPAPIERTISKPK
**SEQ ID NO: 35** amino acid sequence of constant CH3 domain of mAb1-VH 
GSVRAPQVYVLPPPEEEMTKKQVTLTCMVTDFMPEDIYVEWTNNGKTELNYKNTEP
VLDSDGSYFMYSKLRVEKKNWVERNSYSCSVVHEGLHNHHTTKSFSRTPGK
**SEQ ID NO: 36** amino acid sequence of hinge region between CH1 and CH2 of mAb1-VH
   EPRGPTIKPCPPCKCP
**SEQ ID NO: 37** human amino acid sequence of PLA2G1B 
MKLLVLAVLL TVAAADSGIS PRAVWQFRKM IKCVIPGSDP FLEYNNYGCY
CGLGGSGTPV DELDKCCQTH DNCYDQAKKL DSCKFLLDNP YTHTYSYSCS
GSAITCSSKN KECEAFICNC DRNAAICFSK APYNKAHKNL DTKKYCQS
**SEQ ID NO: 38** macaque amino acid sequence of PLA2G1B

MKLLVLAVLL TVAAAESGIS SRAVWQFRKL IKCVIPGSDP YLEYNNYGCY
CGLGGSGTPV DELDKCCQTH DNCYDQAKKL SSCKFLLDNP YTHTYSYSCS
NSEITCSSKN KECEAFICNC DRNAAICFSK APYNKEHKNL DSKKYCQS

## Claims

1. A monoclonal antibody, or an antigen-binding fragment thereof, wherein said antibody or fragment thereof comprises a light chain variable region comprising (i) a CDR1 comprising, consisting, or consisting essentially of SEQ ID NO: 21, and/or (ii) a CDR2 comprising, consisting, or consisting essentially of SEQ ID NO: 22, and/or (iii) a CDR3 comprising, consisting, or consisting essentially of SEQ ID NO: 23.

2. A monoclonal antibody, or an antigen-binding fragment thereof, wherein said antibody or fragment thereof comprises a heavy chain variable region comprising (i) a CDR1 comprising, consisting, or consisting essentially of SEQ ID NO: 24, and/or (ii) a CDR2 comprising, consisting, or consisting essentially of SEQ ID NO: 25, and/or (iii) a CDR3 comprising, consisting, or consisting essentially of SEQ ID NO: 26.

3. A monoclonal antibody according to claim 1 or 2, wherein said antibody comprises a light chain variable region comprising SEQ ID NO: 1 and/or a heavy chain variable region comprising SEQ ID NO: 5.

4. A monoclonal antibody according to any one of claims 1 to 3, wherein said antibody comprises a light chain sequence of SEQ ID NO: 18, and/or a heavy chain sequence of SEQ ID NO: 20.

5. A monoclonal antibody or fragment according to any one of the preceding claims, wherein said antibody or fragment binds a mammalian PLA2G1B protein, preferably human or simian PLA2G1B protein.

6. A monoclonal antibody or fragment according to any one of the preceding claims, wherein said antibody or fragment inhibits PLA2G1B.

7. A monoclonal antibody or an antigen-binding fragment thereof, wherein said antibody or fragment competes with an antibody of claim 4 for binding to a mammalian PLA2G1B.

8. A monoclonal antibody according to any one of the preceding claims, for use in the treatment of an immunodeficiency or an associated disease.

9. A monoclonal antibody according to any one of the preceding claims, for use to restore CD4 T-cells function in a HIV-, SIV- or SHIV-infected subject.

10. A monoclonal antibody for use according to claim 8 or 9, wherein said antibody comprises:
(i) a light chain comprising SEQ ID NO: 1 or 18 and/or a heavy chain comprising SEQ ID NO: 5 or 20 ; or
(ii) a light chain comprising SEQ ID NO: 9 and/or a heavy chain comprising SEQ ID NO: 13.

11. A composition comprising an antibody or fragment of any one of claims 1 to 7.

12. A nucleic acid encoding an antibody of any one of claims 1 to 7, or a light or heavy chain thereof, or a variable domain thereof.

13. The nucleic acid of claim 12, comprising a sequence of any one of SEQ ID NO: 3, 7, 11, 15, 17 or 19.

14. A method for detecting mammalian PLA2G1B protein in a sample, comprising:
(i) contacting the sample with a monoclonal antibody of any one of claims 1 to 7, and
(ii) detecting the presence of an antibody-antigen complex formed in step (i).

15. A kit comprising a least one antibody, or a fragment thereof, according to any one of claims 1 to 7, and a reagent to perform, detect or quantify an immune reaction, or an antibody-antigen complex.
